# EUROPEAN PATENT SPECIFICATION

(11) **EP 1 463 803 B1**
(45) Date of publication and mention of the grant of the patent: **14.02.2018**
(21) Application number: 02804740.5
(22) Date of filing: 06.12.2002
(51) Int. Cl.: C12N 5/04, C12N 5/10, A01N 63/00, A61K 48/00, A61K 35/12, C12N 5/078

(54) **HEMATOPOIETIC CELLS FROM HUMAN EMBRYONIC STEM CELLS**
HÄMATOPOETISCHE ZELLEN VON MENSCHLICHEN EMBRYONALEN STAMMZELLEN
OBTENTION DE CELLULES HEMATOPOIETIQUES A PARTIR DE CELLULES SOUCHES EMBRYONNAIRES HUMAINES

(30) Priority: 07.12.2001 US 338979 P
(43) Date of publication of application: 06.10.2004
(60) Divisional application: 10175120.4
(73) Proprietor: Asterias Biotherapeutics, Inc., Fremont CA 94555 (US); The University of Western Ontario, London, ON N6A 5K8 (CA)
(72) Inventor: BHATIA, Mickie, London, Ontario N6G 4M8 (CA); MADRENAS, Joaquin, London, Ontario N5Y 4G9 (CA); FERBER, Iris, A., San Jose, CA 95128-3693 (US); MAJUMDAR, Anish, Sen, Sunnyvale, CA 94087 (US)
(74) Representative: Bassil, Nicholas Charles
(86) International application number: PCT/US2002/039091
(87) International publication number: WO 2003/050251

(56) References cited:
- WO-A-01/34776
- LI F ET AL: "BONE MORPHOGENETIC PROTEIN 4 INDUCES EFFICIENT HEMATOPOIETIC DIFFERENTIATION OF RHESUS MONKEY EMBRYONIC STEM CELLS IN VITRO" BLOOD, W.B.SAUNDERS COMPANY, ORLANDO, FL, US, vol. 98, no. 2, 15 July 2001 (2001-07-15), pages 335-342, XP001181131 ISSN: 0006-4971
- KAUFMAN DAN S ET AL: "Hematopoietic colony-forming cells derived from human embryonic stem cells" PROCEEDINGS OF THE NATIONAL ACADEMY OF SCIENCES OF THE UNITED STATES OF AMERICA, vol. 98, no. 19, 11 September 2001 (2001-09-11), pages 10716-10721, XP002308712 ISSN: 0027-8424
- KAUFMAN D S ET AL: "HUMAN ES CELLS - HAEMATOPOIESIS AND TRANSPLANTATION STRATEGIES" JOURNAL OF ANATOMY, CAMBRIDGE UNIVERSITY PRESS, CAMBRIDGE,, GB, vol. 200, no. PART 3, March 2002 (2002-03), pages 243-248, XP001104907 ISSN: 0021-8782
- ZHAN X ET AL: "Functional antigen-presenting leucocytes derived from human embryonic stem cells in vitro" LANCET THE, LANCET LIMITED. LONDON, GB, vol. 364, no. 9429, 10 July 2004 (2004-07-10), pages 163-171, XP004520463 ISSN: 0140-6736
- KEHAT I. ET AL.: 'Long term, high resoluation electrophysiological assessment of human embryonic stem cell derived cardiomyocytes a novel in vitro model for the human heart' CIRCULATION vol. 102, no. 18, SUPPL. II, 13 October 2000, page II-4, ABSTRACT NO. 6, XP002964185
- FIRPO M.T. ET AL.: 'Controlled differentiation of human embryonic stem cells' DEVELOPMENTAL BIOLOGY vol. 222, no. 1, 01 June 2000, page 234, ABSTRACT NO. 69, XP002964186
- THOMSON J.A. ET AL.: 'Human embryonic stem cell and embryonic germ cell lines' TRENDS IN BIOTECHNOLOGY vol. 18, no. 1, 2000, pages 53 - 57, XP000882397
- SCHULDINER M. ET AL.: 'Effects of eight growth factors on the differentiation of cell derived from human embryonic stem cells' PROC. NATL. ACAD. SCI. USA vol. 97, no. 21, 10 October 2000, pages 11307 - 11312, XP002939896
- LING V. ET AL.: 'In vitro differentiation of embryonic stem cells: Immunophenotypic analysis of cultured embryoid bodies' JOURNAL OF CELLULAR PHYSIOLGY vol. 171, 1997, pages 104 - 115, XP002964187
- KAUFMAN D. ET AL.: 'Directed differentiation of human embryonic stem cells into hematopoietic colony forming cells' BLOOD vol. 94, no. 10, SUPPL. I, PT. 1, 15 November 1999, page 34A, ABSTRACT NO. 138, XP000942815

## Description

### TECHNICAL FIELD

This invention relates generally to the fields of cell biology, embryonic stem cells, and cell differentiation. More specifically, this invention provides a growth environment suitable for producing differentiated cells with hematopoietic potential for use in drug development and transplantation therapy.

### BACKGROUND

Leukemia is a cancer of blood forming cells with a grim prognosis. The Leukemia Society of America estimates that 28,700 people in the U.S. were diagnosed with leukemia in 1998. Considerable progress has been made in the last decade to treat leukemia with allogeneic or autologous hematopoietic stem cells, in conjunction with radiation or chemotherapy. Autologous transplants are also used in the treatment of late stage breast, ovarian, and prostate cancer. Stem cell transplantation is currently being tested in clinical trials as a treatment for severe life-threatening autoimmune disorders.

Unfortunately, suitable hematopoietic stem cells are often not available for the treatment of these conditions. Allogeneic cells from another donor are difficult to match, which has led to development of autologous donations, where the therapeutic cells are derived from the patient's own bone marrow. Autologous donations require time to prepare enough cells to transplant, and there is always the risk that the cancer will be reintroduced to the patient with the administered cells.

A good deal of research has been done to characterize the stem cells present in human blood and bone marrow that are believed to replenish the hematopoietic system on an ongoing basis. Gunsilius et al. (Biomed. Pharmacother. 55:186, 2001) provide a general review. U.S. Patent 5,750,397 reports cultures of human hematopoietic stem cells that are CD34 +ve and capable of proliferation and differentiation, derived from human bone marrow samples. U.S. Patent 5,192,553 reports isolation of fetal and neonatal stem and progenitor cells of the blood. U.S. Patent 5,635,386 reports methods for regulating specific cell lineages in a human hematopoietic cell culture. European patent publication EP 455,482 A3 reports a subset of human progenitor cells lacking CD38 but expressing CD34.

Vaziri et al. (Proc. Natl. Acad. Sci. USA 91:9857, 1994) report the loss of telomeric DNA as human hematopoietic stem cells age. Chiu et al. (Geron Corporation; Stem Cells 14:239, 1996) describe differential expression of telomerase activity in hematopoietic progenitors from adult human bone marrow. Gaffney et al. (Blood 91:1662, 1998) report the effect of Flt-3 ligand and bone marrow stroma-derived factors on primary human CD34 +ve marrow progenitors. Koller et al. (J. Hematother. 5:449, 1996) compare the effect of Flt-3 ligand and c-kit as stimulators of ex vivo hematopoietic cell expansion.

Bhatia et al. (Proc. Natl. Acad. Sci 94:5320, 1997) reported purification pf primitive human hematopoietic cells capable of repopulating immune deficient mice. Bhatia et al. (Nature Med. 4:1038, 1998) reported a class of human hematopoietic cells with SCID repopulating activity. Gallacher et al. (Blood 96:1, 2000) reported isolation of novel circulating human embryonic blood stem cells. International Patent Publication WO 99/23205 claims a substantially homogeneous population of human hematopoietic stem cells that are CD34 negative and Lin negative. Karanu et al. (J. Exp. Med. 192:1365, 2000) reported the Notch ligand Jagged-1 as a growth factor for hematopoietic stem cells. Bhatia et al. (J. Exp. Med. 189:1139, 1999) reported that bone morphogenetic proteins regulate the developmental program of human hematopoietic stem cells. Karanu et al. (Blood 97:1960, 2001) reported that Delta-2 and Delta-4 function as mitogenic regulators of primitive human hematopoietic cells. Bhardwaj et al. (Nature Immunol 2:172, 2001) reported that the factor sonic hedgehog induces proliferation of human hematopoietic cells.

The important hematopoietic progenitors from human bone marrow and cord blood have been identified, and effective ways have been discovered to manipulate them in vitro. But the paucity of these cells as a percentage of the donated human cell population remains a problem.

An alternative source is pluripotent cells isolated from early embryonic tissue. Techniques have been developed recently to isolate and culture human ES cells (Thomson et al., Science 282:114, 1998; U.S. Patents 6,090,622 & 6,200,806) and human embryonic germ cells (Shamblott et al., Proc. Natl. Acad. Sci. USA 95:13726, 1998; U.S. Patent 6,090,622). International Patent Publications WO 99/20741 and WO 01/51616 (Geron Corp.) provide methods and materials for growing primate-derived primordial stem cells in feeder-free culture, which considerably facilitates the preparation of these cells and their derivatives for human therapy.

Preliminary efforts to differentiate human pluripotent stem cells into cells of the hematopoietic lineage have been reported by Li et al. (Blood 15:98, 2001); U.S. Patent 6,280,718 (Wisconsin); and Kaufman et al. (Proc. Natl. Acad. Sci. USA 98:10716, 2001b). Coculturing with murine bone marrow cells or yolk sac endothelial cells was necessary in order to generate cells with hematopoietic markers.

For embryonic stem cell derived hematopoietic cells to become a commercially viable proposition, there is a need to develop new procedures that eliminate the need for coculturing with stromal cells, and that provide a substantially improved yield compared with cells available from bone marrow.

### SUMMARY

The present invention provides a growth environment for efficient production of primate cells that have differentiated from pluripotent cells into cells of the hematopoiesis lineage. According to a first aspect of the invention, there is provided a growth environment comprising a culture medium and a substrate on a solid surface for differentiating pPS cells into hematopoietic cells, wherein the culture medium contains a mixture of hematopoietic growth factors, chosen from two or more of: stem cell factor, interleukin-3, interleukin-6, granulocyte-colony stimulating factor, BMP-2, BMP-4, BMP-7, sonic hedgehog, Delta-1, Jagged-1 and thrombopoietin, wherein no stromal cells are present, and wherein the growth environment does not comprise pPS cells. The mixture of hematopoietic growth factors may comprise at least two of the following: stem cell factor (SCF), FLT-3 ligand, IL-3, IL-6, and granulocyte colony stimulating factor (G-CSF). The growth environment may be suitable for producing hematopoietic progenitors, erythroid cells, granulocytic cells, monocytic cells, megakaryocytes, or lymphoid cells. The pPS cells may be human embryonic stem cells or cultured from a human blastocyst.

Populations of cells are described that are considerably enriched for hematopoietic progenitor cells. In turn, the hematopoietic progenitors can be further differentiated into colonies of erythroid, granulocytic, monocytic, megakaryocyte, and lymphoid cell lines. The compositions, methods, and techniques described in this disclosure hold considerable promise for a variety of applications, including drug screening and various forms of clinical therapy.

Also described is a population that proliferates in culture and has certain features characteristic of hematopoietic cells. The cell population is obtained by differentiating primate pluripotent stem (pPS) cells, exemplified by an established line of human embryonic stem cells. Included are populations in which at least 1% of the cells are CD45 +ve, have other markers characteristic of hematopoietic cells listed below, and have a minimal proportion of undifferentiated pPS cells. The cell populations may form colonies in a methyl-cellulose assay for hematopoietic colony forming units (CFU) at a high plating efficiency, which may in turn form secondary colonies when replated in a second CFU assay. When injected into NOD-SCID mice, the cells may form circulating erythroid cells, granulocytic cells, monocytes, megakaryocytes, or lymphoid cells. Included are cells that have been genetically altered to express a heterologous gene for purposes of gene therapy, or to extend cell replicative capacity.

Also described herein is a population of human hematopoietic cells that have at least one of the characteristics described in this disclosure, for example: at least -20% of the cells express CD34 from an endogenous gene; at least -2% of the cells express CD45 from an endogenous gene; or wherein the cells form colonies in a CFU assay at high plating efficiency. This covers human cell compositions made by any process including but not limited to differentiation of human pluripotent stem cells, or any other process that does not involve cell separation using specific antibody (such as an anti-CD34 antibody) or its equivalent.

Also described herein is a method for making hematopoietic cells by differentiating pPS cells. For example, pPS cells can be harvested from a feeder-free culture, and then initiated into the differentiation pathway by forming embryoid bodies or by some other means. Then the initiated cells can be cultured with a mixture of hematopoietic growth factors, thereby obtaining cells that form colonies in a CFU assay. The mixture of hematopoietic growth factors can contain one or more of the following hematopoietic differentiation factors: stem cell factor (SCF), FLT-3 ligand, IL-3, IL-6, G-CSF, sonic hedgehog, or other cytokines listed in this disclosure, possibly in combination with a bone morphogenic protein such as BMP-4. Coculturing with foreign stromal cells or any other cells having a different genome is usually not necessary. The method can be used to produce hematopoietic progenitors, or mature hematopoietic cells such as erythroid cells, granulocytic cells, monocytic cells, megakaryocytes, or lymphoid cells.

### DRAWINGS

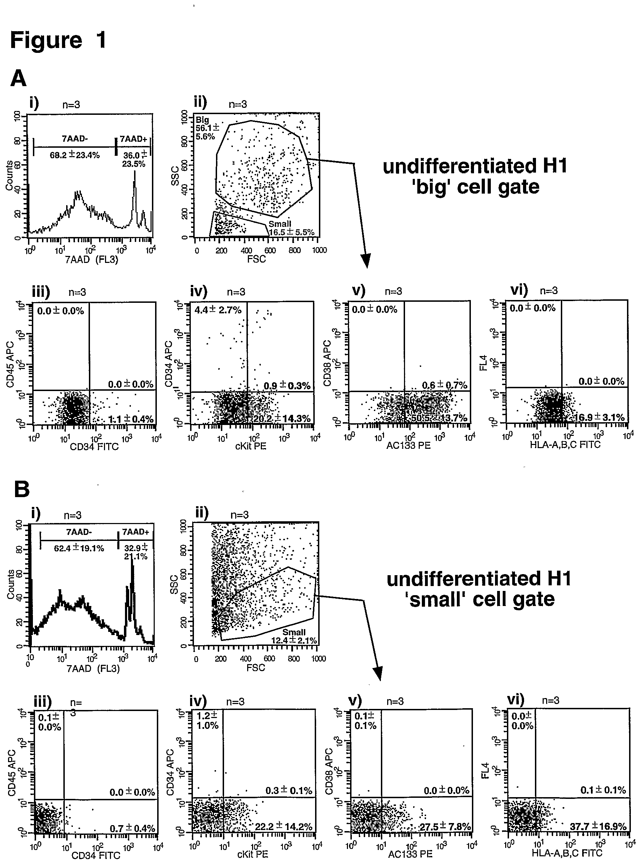

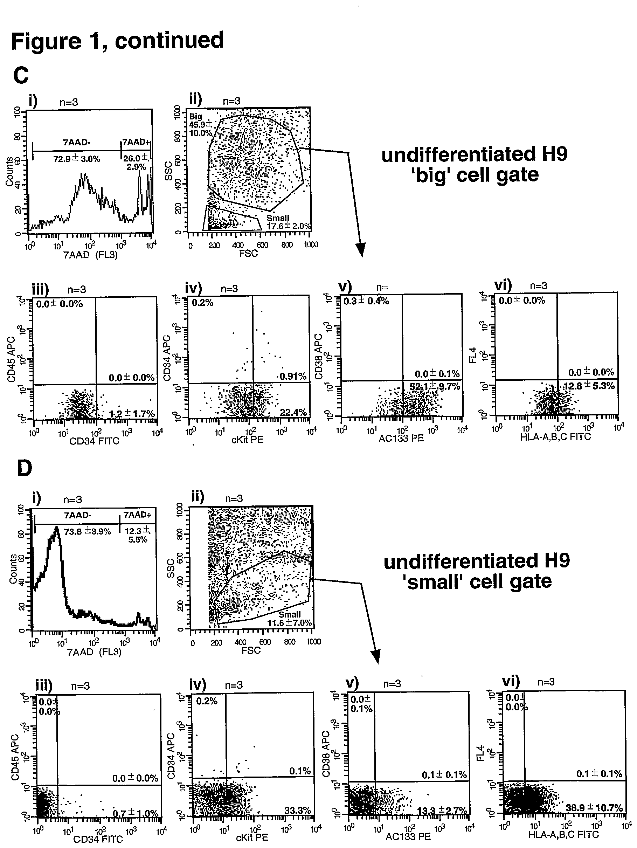

**Figure 1** shows flow cytometry analysis of undifferentiated human embryonic stem (hES) cells. Cells were gated for viability (7AAD -ve; panel i) and size (ii), and then for expression of hematopoietic cell surface markers (iii-vi) in undifferentiated ES cell populations. None of the cells expressed the human hematopoietic marker CD45, and only 1.2% were CD34 +ve (a marker of primitive human hematopoietic cells).

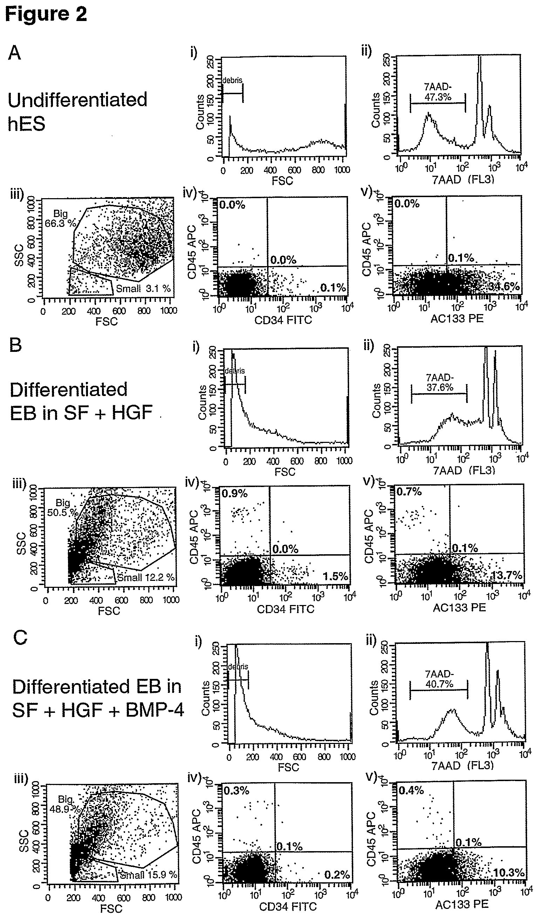

**Figure** 2 shows flow cytometry analysis of hematopoietic cells obtained by differentiating the H( line of hES cells. Differentiation was initiated by growing strips of hES cells as aggregates in medium containing 20% FBS for 10 days. The cells were then cultured in a serum-free medium (SF) containing hematopoietic growth factors (HGF, which were SCF, Flt-3 ligand, IL-3, IL-6, and G-CSF) with or without bone morphogenic protein 4 (BMP-4). The CD45 marker identifies hematopoietic progenitor cells.

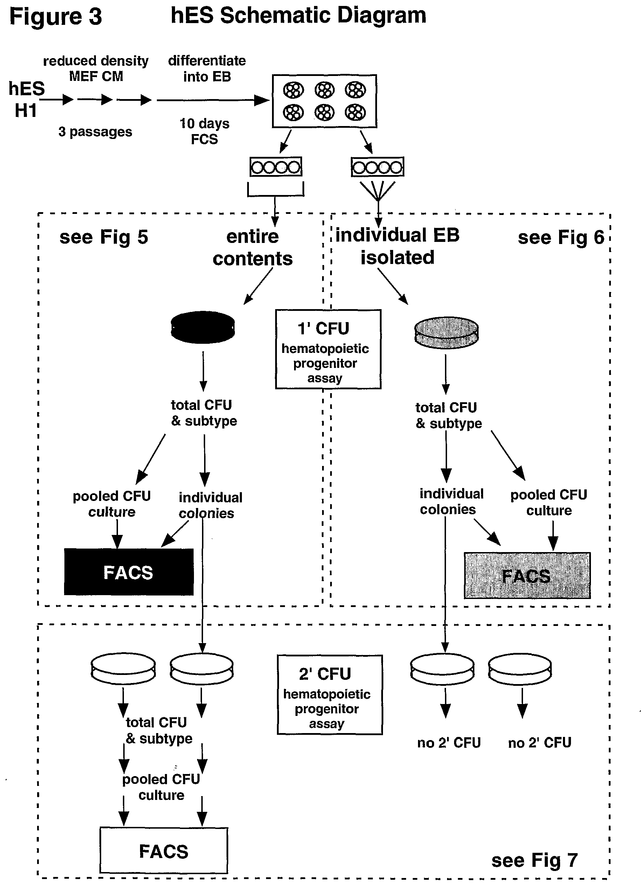

**Figure 3** is a scheme in which the H1 line of hES cells was differentiated into hematopoietic progenitors. After differentiation in FCS containing medium, the entire culture (left) or individual embryoid bodies (right) were placed in a colony forming (CFU) assay in methylcellulose containing stem cell factor, GM-CSF, IL-3, and EPO. Colonies formed were characterized for hematopoietic phenotype by flow cytometry, and passaged into a secondary CFU assay.

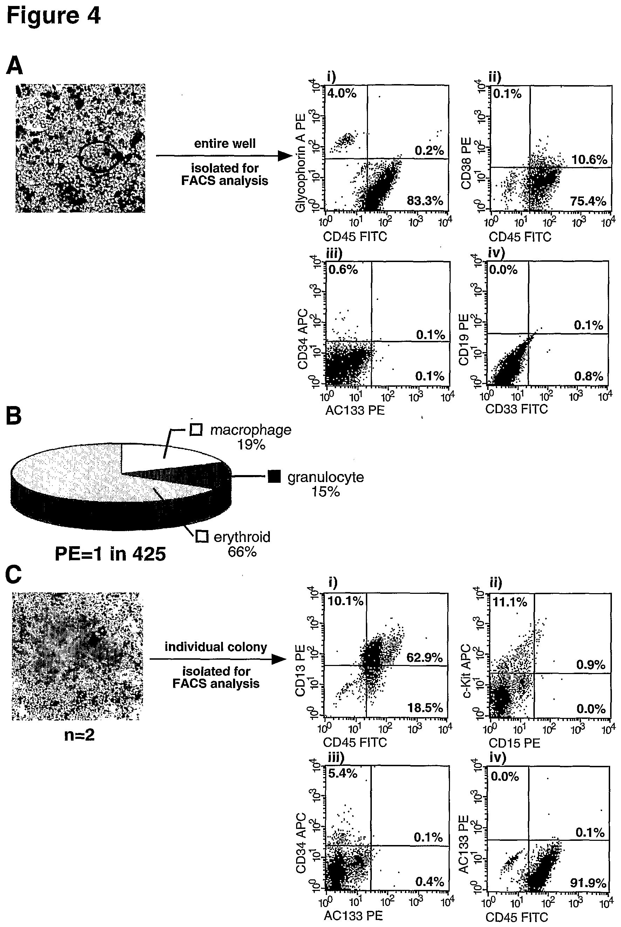

**Figure 4** shows hematopoietic cells formed from the entire embryoid body culture, according to the scheme on the left side of Figure 3. When the entire CFU assay was analyzed (Panel A), 83-86% stained for CD45, confirming the presence of hematopoietic cells, and 4% stained for glycophorin A (4%) confirming the presence of erythroid cells. Morphology assessment is shown in Panel B. 47 colonies were produced from 20,000 input cells, a plating efficiency of 1 in 425. The colony shown in Panel C was picked for marker analysis, 81-92% of the cells were CD45 +ve, and 73% were CD13 +ve.

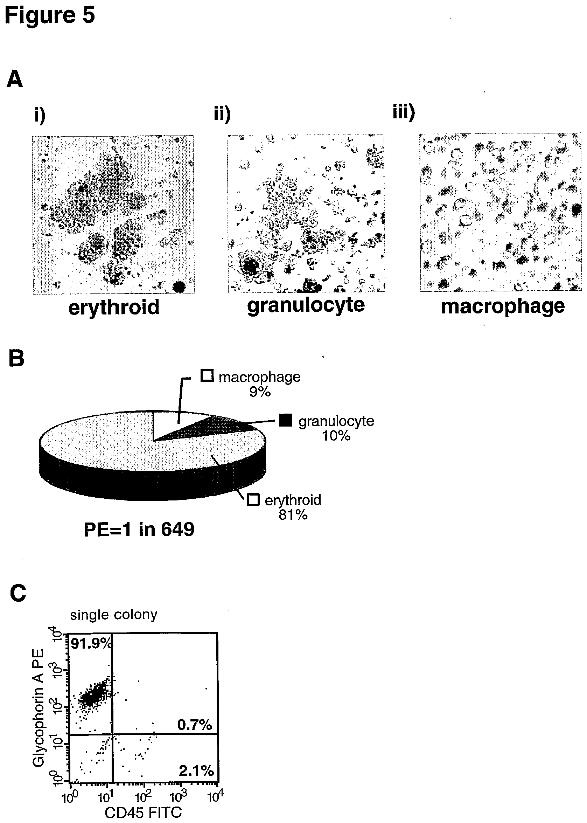

**Figure 5** shows hematopoietic cells formed from isolated embryoid bodies, according to the scheme on the right side of Figure 3. Colonies of erythroid cells, granulocytic cells, and macrophages were all identified in the CFU assay. Two erythroid colonies were analyzed by flow cytometry, and were found to be 93% glycophorin A positive.

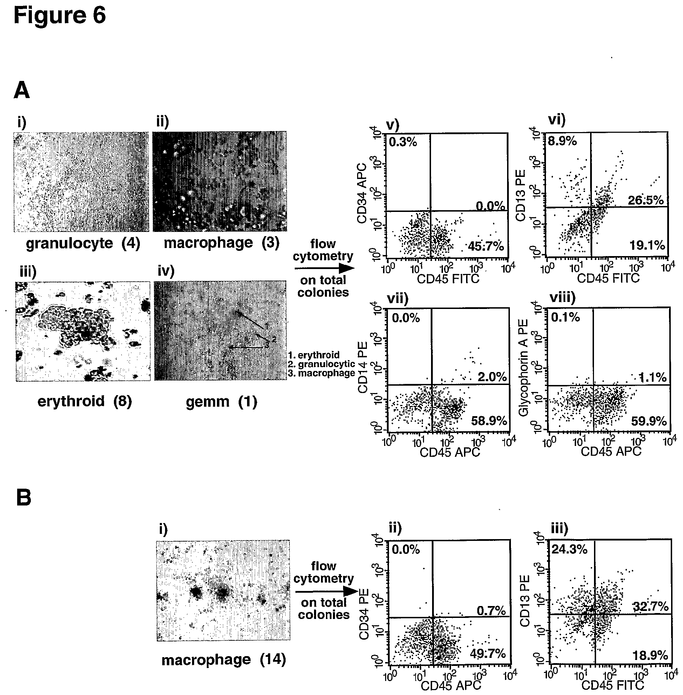

**Figure 6** shows what happens when two colonies picked from the CFU assay shown in Figure 3 were replated in a secondary CFU assay. Panel A shows the different secondary colonies derived from a primary granulocytic colony containing 82,500 cells (numbers of each colony type are shown below). The secondary colonies had features of granulocytic cells, macrophages, erythroid cells, and a GEMM colony (a mixture of hematopoietic cell types). There was a high level of CD45 and CD13 expression, but low levels of CD34 and CD14. Another primary granulocytic colony (12,500 cells) was passaged into the secondary CFU assay (Panel B) and formed 14 colonies, all with characteristics of monocytic cells.

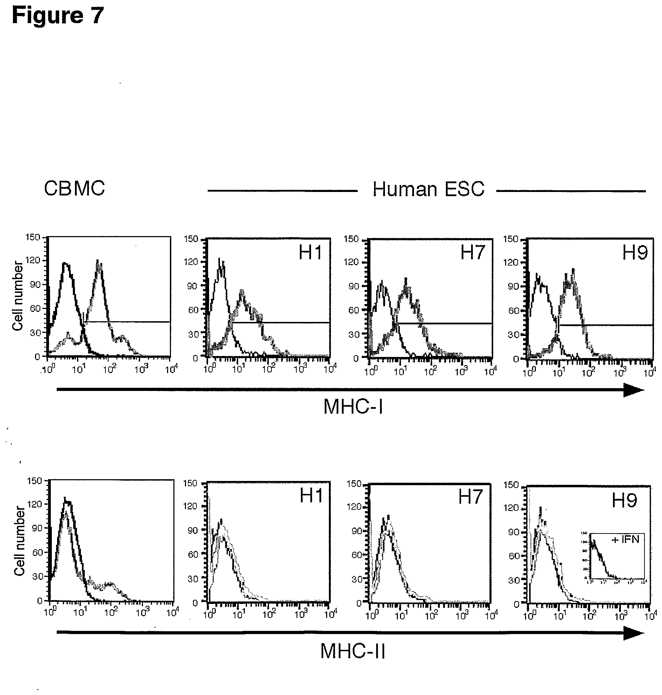

**Figure 7** shows the expression of major histocompatibility complex (MHC) Class I and Class II antigens on cord blood mononuclear cells (CBMC), and undifferentiated hES cell lines H1, H7, and H9. Grey line indicates staining for MHC staining; the solid line indicates antibody control. The undifferentiated hES cells were positive for MHC Class I, but not Class II - even after treatment with interferon γ (inset).

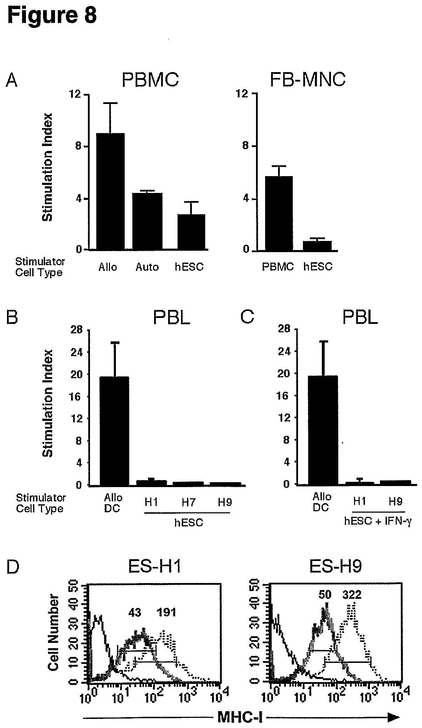

**Figure 8** shows the effect of undifferentiated hES cells in a mixed lymphocyte reaction. In Panel A, hES cells failed to stimulate proliferation of allogeneic peripheral blood or cord blood mononuclear cells. In Panel B, all three hES cell lines failed to stimulate proliferation, even after enrichment of the responding population for T cells by monocyte depletion. In Panel C, hES cells were prepared by culturing with IFN-γ to increase MHC Class I expression, but still failed to stimulate proliferation of the T cells.

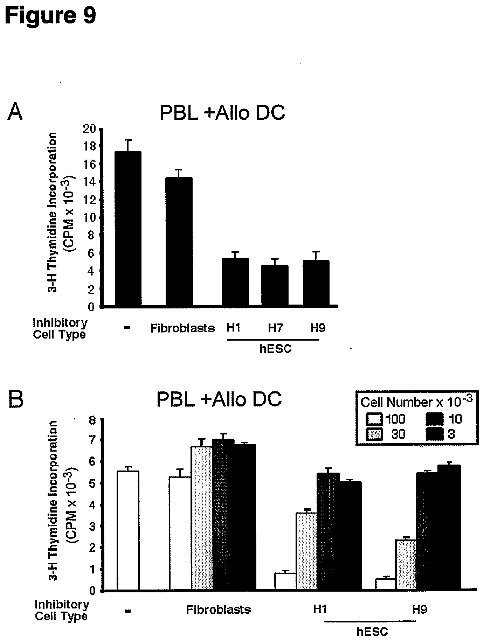

**Figure 9** shows that hES cells are also able to inhibit a mixed lymphocyte reaction stimulated by third-party antigen-presenting cells. In Panel A, a vigorous proliferative response was observed when T cells were stimulated by allogeneic dendritic cells (DC). Adding human fibroblasts to the culture had minimal effect, but adding undifferentiated hES cells abrogated the response. In Panel B, the inhibitory effect is shown to be dependent on the number of hES cells present in the MLR. The reaction was significantly inhibited by as few as 3 × 10⁴ hES cells.

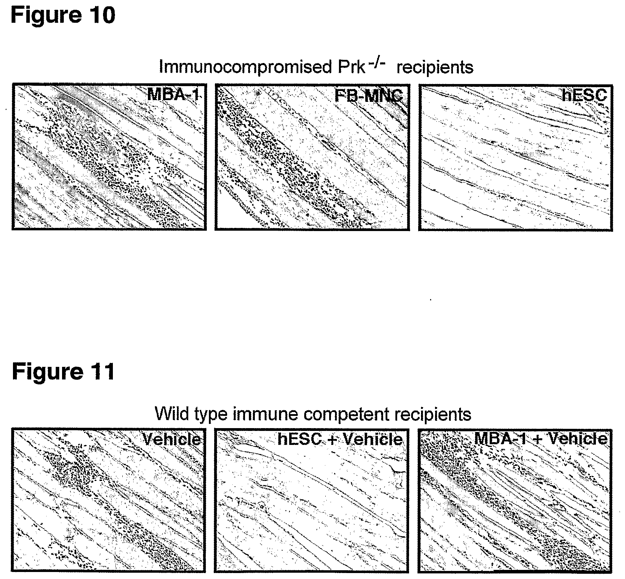

**Figure 10** shows the response generated by injection of cells into immunodeficient Prk-/- SCID mice. Both the MBA-1 stromal cells and the fetal mononuclear cells were able to induce a granulocytic infiltration response, but undifferentiated hES cells had no observed effect.
**Figure 11** shows the response generated by injection of cells into wild-type CD-1 mice. Injection of endotoxin containing PBS alone induced lymphocyte and granulocyte infiltration at the injection site. However, injection of vehicle together with hES cells completely abrogated leukocyte infiltration (right), whereas MBA-1 cells failed to inhibit infiltration (inset). Undifferentiated hES cells are apparently unable to induce a rejection response in this situation, and they prevent host cell infiltration at the injection site, which demonstrates an ability to inhibit inflammation.

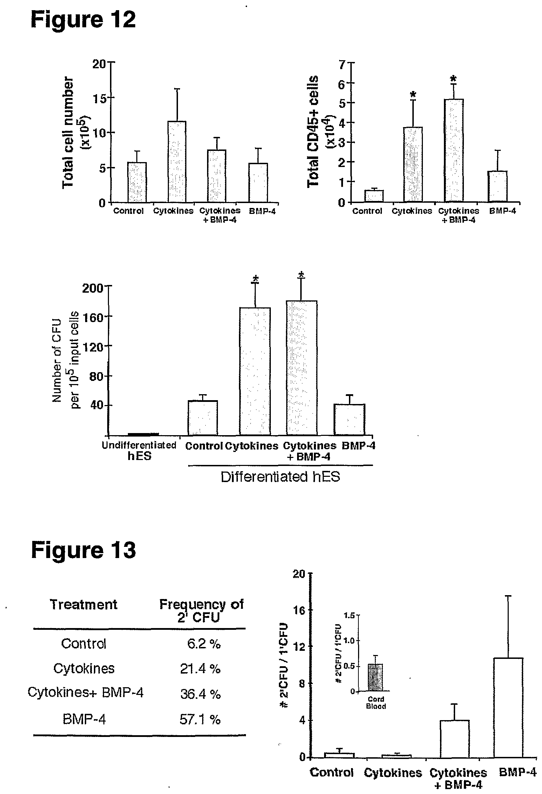

**Figure 12** shows phenotypic and functional features of hematopoietic cells obtained by culturing hPS cells in cytokines and/or BMP-4 the next day after forming embryoid bodies. The cytokines improve the total cell yield, and considerably enhance the proportion of CD45 +ve cells, and cells that generate CFUs.
**Figure 13** shows the results of secondary CFUs, emphasizing the importance of BMP-4 during the initial differentiation process. Hematopoietic cells made using BMP-4 (with or without cytokines) produced a high proportion of secondary colonies. This demonstrates that differentiating hES cells in the presence of BMP-4 produces hematopoietic progenitors having considerable self-renewal capacity.

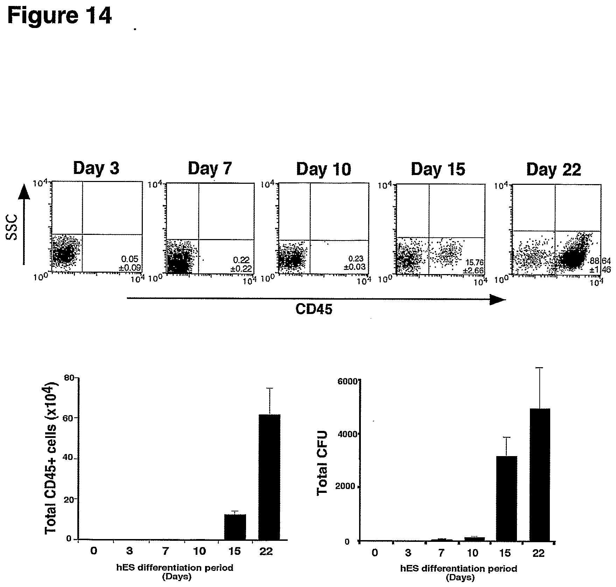

**Figure 14** shows the results of a protocol in which the kinetics of cell phenotype and function was followed during the differentiation process. CD45 +ve cells emerged by Day 15, and increased considerably by Day 22. Clonogenic activity was high by Day 15, and the increase on Day 22 was not significant. Under these conditions, the first 15 days may represent the critical window for the cytokines and BMP to direct hematopoietic differentiation.

### DETAILED DESCRIPTION

This invention as defined in the claims solves the problem of generating large populations of human hematopoietic cells by showing how to efficiently differentiate them from pluripotent stem cells.

It has been discovered that human embryonic stem cells can be coaxed along the hematopoiesis differentiation pathway by initiating differentiation in a non-specific fashion, and then culturing the initiated cells in a cocktail of differentiation factors. Different combinations of growth factors are effective to promote hematopoietic cells. A particularly effective combination includes stem cell factor (SCF), Flt-3 ligand, IL-3, IL-6, and G-CSF. Culturing in this cocktail for an appropriate period generates a population considerably enriched for hematopoietic precursor cells, which are multipotent for the various hematopoietic cell lineages, and proliferate actively in culture. In turn, the hematopoietic precursors can be driven further down the myeloid differentiation pathway by culturing with SCF, GM-CSF, IL-3, and erythropoietin (EPO).

Unlike what was reported by Kaufman et al. (supra), this disclosure establishes that coculturing with stromal cells is not a necessary part of performing the derivation.

To the contrary, using the techniques in this disclosure, it is possible to generate populations of differentiated cells that are considerably enriched for the hematopoietic phenotype. By including both cytokines and bone morphogenic protein 4 (BMP-4) in the differentiation cocktail, cell populations have been obtained that contain 8% CD45 +ve cells (a marker for multipotent hematopoietic cells) and 22% CD34 +ve cells (a marker for primitive hematopoietic progenitors). Remarkably, over 5% of the cells are double positive for CD45 and CD34. The presence of the CD45 marker correlates with active colony forming cells as measured in a CFU assay. Hematopoietic cells derived from embryonic stem cells produce colonies at a very high plating efficiency.

This discovery is important, because it provides hematopoietic cell populations that appear to contain more hematopoietic progenitors than is apparently obtainable from any current source - including peripheral blood, adult bone marrow, or even cord blood. Starting populations of 1 × 10⁵ hES cells differentiated with cytokines yield at least -137 hematopoietic progenitors, comparable with human cord blood (182) or mobilized bone marrow progenitors in peripheral blood (249). Since human embryonic stem cells can be caused to proliferate indefinitely, this invention provides a system that can be used to generate unbounded quantities of hematopoietic progenitors - and progeny that are committed to one of the hematopoietic subtypes, or have differentiated to mature erythrocytes or leukocytes.

The disclosure that follows provides further information on the production and testing of hematopoietic cells of this invention. It also provides extensive illustrations of how these cells can be used in research, pharmaceutical development, and the therapeutic management of blood-related abnormalities.

### Definitions

For purposes of this disclosure, the term "hematopoietic cell" refers to any cell from the hematopoiesis pathway. The cell expresses some of the accepted morphological features and phenotypic markers (exemplified below) that are characteristic of the hematopoietic lineage. Included are hematopoietic progenitors, committed replication-competent or colony forming cells, and fully differentiated cells.

A "hematopoietic progenitor", "hematopoietic precursor" or "hematopoietic stem cell" is a cell that has the capability to generate fully differentiated hematopoietic cells, and has the capability to self-renew. Typically, it does not produce progeny of other embryonic germ layers when cultured by itself in vitro, unless dedifferentiated or reprogrammed in some fashion.

In the context of cell ontogeny, the adjective "differentiated" is a relative term. A "differentiated cell" is a cell that has progressed further down the developmental pathway than the cell it is being compared with. Thus, pluripotent embryonic stem cells can differentiate to lineage-restricted precursor cells, such as a multipotent hematopoietic progenitor, that has the capacity to form cells of each of the erythroid, granulocytic, monocyte, megakaryocyte, and lymphoid lines. These progenitors can further differentiate into self-renewing cells that are committed to form cells of only one of these four hematopoietic lines. These in turn can be differentiated further to an end-stage differentiated cell, which plays a characteristic role, and may or may not retain the capacity to proliferate further. Erythrocytes, monocytes, macrophages, neutrophils, eosinophils, basophils, platelets, and lymphocytes are examples of terminally differentiated cells.

A "differentiation agent", as used in this disclosure, refers to one of a collection of compounds that are used in culture systems of this invention to produce differentiated cells of the hematopoietic lineage (including precursor cells and terminally differentiated cells). No limitation is intended as to the mode of action of the compound. For example, the agent may assist the differentiation process by inducing or assisting a change in phenotype, promoting growth of cells with a particular phenotype or retarding the growth of others, or acting in concert with other agents through unknown mechanisms.

Prototype "primate Pluripotent Stem cells" (pPS cells) are pluripotent cells derived from pre-embryonic, embryonic, or fetal tissue at any time after fertilization, and have the characteristic of being capable under appropriate conditions of producing progeny of several different cell types that are derivatives of all of the three germinal layers (endoderm, mesoderm, and ectoderm), according to a standard art-accepted test, such as the ability to form a teratoma in 8-12 week old SCID mice. The term includes both established lines of stem cells of various kinds, and cells obtained from primary tissue that are pluripotent in the manner described.

Included in the definition of pPS cells are embryonic cells of various types, exemplified by human embryonic stem (hES) cells, described by Thomson et al. (Science 282:1145, 1998); embryonic stem cells from other primates, such as Rhesus stem cells (Thomson et al., Proc. Natl. Acad. Sci. USA 92:7844, 1995), marmoset stem cells (Thomson et al., Biol. Reprod. 55:254, 1996) and human embryonic germ (hEG) cells (Shamblott et al., Proc. Natl. Acad. Sci. USA 95:13726, 1998). Other types of pluripotent cells are also included in the term. Any cells of primate origin that are capable of producing progeny that are derivatives of all three germinal layers are included, regardless of whether they were derived from embryonic tissue, fetal tissue, or other sources. The pPS cells are preferably not derived from a malignant source. It is desirable (but not always necessary) that the cells be karyotypically normal.

pPS cell cultures are described as "undifferentiated" when a substantial proportion of stem cells and their derivatives in the population display morphological characteristics of undifferentiated cells, clearly distinguishing them from differentiated cells of embryo or adult origin. Undifferentiated pPS cells are easily recognized by those skilled in the art, and typically appear in the two dimensions of a microscopic view in colonies of cells with high nuclear/cytoplasmic ratios and prominent nucleoli. It is understood that colonies of undifferentiated cells within the population will often be surrounded by neighboring cells that are differentiated.

"Feeder cells" are terms used to describe cells of one type that are co-cultured with cells of another type, to provide an environment in which the cells of the second type can grow. Certain types of pPS cells can be supported by primary mouse embryonic fibroblasts, immortalized mouse embryonic fibroblasts, or human fibroblast-like cells differentiated from hES cell. pPS cell populations are said to be "essentially free" of feeder cells if the cells have been grown through at least one round after splitting in which fresh feeder cells are not added to support growth of the pPS cells.

The term "embryoid bodies" is a term of art synonymous with "aggregate bodies", referring to aggregates of differentiated and undifferentiated cells of various size that appear when pPS cells overgrow in monolayer cultures, or are maintained in suspension cultures. Embryoid bodies are a mixture of different cell types, typically from several germ layers, distinguishable by morphological criteria and cell markers detectable by immunocytochemistry.

A "growth environment" is an environment in which cells of interest will proliferate, differentiate, or mature in vitro. Features of the environment include the medium in which the cells are cultured, any growth factors or differentiation-inducing factors that may be present, and a supporting structure (such as a substrate on a solid surface) if present.

A cell is said to be "genetically altered" or "transfected" when a polynucleotide has been transferred into the cell by any suitable means of artificial manipulation, or where the cell is a progeny of the originally altered cell that has inherited the polynucleotide.

### General Techniques

General methods in molecular genetics and genetic engineering are described in the current editions of Molecular Cloning: A Laboratory Manual, (Sambrook et al., Cold Spring Harbor); Gene Transfer Vectors for Mammalian Cells (Miller & Calos eds.); and Current Protocols in Molecular Biology (F.M. Ausubel et al. eds., Wiley & Sons). Cell biology, protein chemistry, and antibody techniques can be found in Current Protocols in Protein Science (J.E. Colligan et al. eds., Wiley & Sons); Current Protocols in Cell Biology (J.S. Bonifacino et al., Wiley & Sons) and Current protocols in Immunology (J.E. Colligan et al. eds., Wiley & Sons.). Reagents, cloning vectors, and kits for genetic manipulation referred to in this disclosure are available from commercial vendors such as BioRad, Stratagene, Invitrogen, ClonTech, and Sigma-Aldrich Co.

Cell culture methods are described generally in the current edition of Culture of Animal Cells: A Manual of Basic Technique (R.I. Freshney ed., Wiley & Sons); General Techniques of Cell Culture (M.A. Harrison & I.F. Rae, Cambridge Univ. Press), and Embryonic Stem Cells: Methods and Protocols (K. Turksen ed., Humana Press). Tissue culture supplies and reagents are available from commercial vendors such as Gibco/BRL, Nalgene-Nunc International, Sigma Chemical Co., and ICN Biomedicals.

Specialized reference books relevant to this disclosure include Blood Cell Biochemistry, Plenum Pub. Corp. and Kluwer Academic Publishers; Primary Hematopoietic Cells (Human Cell Culture, Vol. 4) by M.R. Koller & B. Palsson eds., Kluwer Academic Publishers, 1999; Molecular Biology of Hematopoiesis and Treatment of Myeloproliferative Diseases: 11th Symposium, Bormio, June 1998 (Acta Haematologica, 101/2) by N.G. Abraham et al. eds., S. Karger Publishing, 1999; The Essential Dracula by B. Stoker, L. Wolf & C. Bing, Penguin Putnam, 1993; and Hematopoiesis: A Developmental Approach by L.I. Zon ed., 1st edition, Oxford University Press, 2001.

### Embryonic Stem Cells

Embryonic stem cells can be isolated from blastocysts of members of the primate species (U.S. Patent 5,843,780; Thomson et al., Proc. Natl. Acad. Sci. USA 92:7844, 1995). Human embryonic stem (hES) cells can be prepared from human blastocyst cells using the techniques described by Thomson et al. (U.S. Patent 6,200,806; Science 282:1145, 1998; Curr. Top. Dev. Biol. 38:133 ff., 1998) and Reubinoff et al, Nature Biotech. 18:399, 2000. Equivalent cell types to hES cells include their pluripotent derivatives, such as primitive ectoderm-like (EPL) cells, as outlined in WO 01/51610 (Bresagen).

hES cells can be obtained from human preimplantation embryos. Alternatively, in vitro fertilized (IVF) embryos can be used, or one-cell human embryos can be expanded to the blastocyst stage (Bongso et al., Hum Reprod 4: 706, 1989). Embryos are cultured to the blastocyst stage in G1.2 and G2.2 medium (Gardner et al., Fertil. Steril. 69:84, 1998). The zona pellucida is removed from developed blastocysts by brief exposure to pronase (Sigma). The inner cell masses are isolated by immunosurgery, in which blastocysts are exposed to a 1:50 dilution of rabbit anti-human spleen cell antiserum for 30 min, then washed for 5 min three times in DMEM, and exposed to a 1:5 dilution of Guinea pig complement (Gibco) for 3 min (Solter et al., Proc. Natl. Acad. Sci. USA 72:5099, 1975). After two further washes in DMEM, lysed trophectoderm cells are removed from the intact inner cell mass (ICM) by gentle pipetting, and the ICM plated on mEF feeder layers.

After 9 to 15 days, inner cell mass-derived outgrowths are dissociated into clumps, either by exposure to calcium and magnesium-free phosphate-buffered saline (PBS) with 1 mM EDTA, by exposure to dispase or trypsin, or by mechanical dissociation with a micropipette; and then replated on mEF in fresh medium. Growing colonies having undifferentiated morphology are individually selected by micropipette, mechanically dissociated into clumps, and replated. ES-like morphology is characterized as compact colonies with apparently high nucleus to cytoplasm ratio and prominent nucleoli. Resulting ES cells are then routinely split every 1-2 weeks by brief trypsinization, exposure to Dulbecco's PBS (containing 2 mM EDTA), exposure to type IV collagenase (∼200 U/mL; Gibco) or by selection of individual colonies by micropipette. Clump sizes of about 50 to 100 cells are optimal.

### Embryonic Germ Cells

Human Embryonic Germ (hEG) cells can be prepared from primordial germ cells present in human fetal material taken about 8-11 weeks after the last menstrual period. Suitable preparation methods are described in Shamblott et al., Proc. Natl. Acad. Sci. USA 95:13726, 1998 and U.S. Patent 6,090,622.

Briefly, genital ridges processed to form disaggregated cells. EG growth medium is DMEM, 4500 mg/L D-glucose, 2200 mg/L mM NaHCO₃; 15% ES qualified fetal calf serum (BRL); 2 mM glutamine (BRL); 1 mM sodium pyruvate (BRL); 1000-2000 U/mL human recombinant leukemia inhibitory factor (LIF, Genzyme); 1-2 ng/mL human recombinant bFGF (Genzyme); and 10 µM forskolin (in 10% DMSO). Ninety-six well tissue culture plates are prepared with a sub-confluent layer of feeder cells (e.g., STO cells, ATCC No. CRL 1503) cultured for 3 days in modified EG growth medium free of LIF, bFGF or forskolin, inactivated with 5000 rad γ-irradiation. ∼0.2 mL of primary germ cell (PGC) suspension is added to each of the wells. The first passage is done after 7-10 days in EG growth medium, transferring each well to one well of a 24-well culture dish previously prepared with irradiated STO mouse fibroblasts. The cells are cultured with daily replacement of medium until cell morphology consistent with EG cells is observed, typically after 7-30 days or 1-4 passages.

### Propagation of pPS Cells in an Undifferentiated State

pPS cells can be propagated continuously in culture, using culture conditions that promote proliferation without promoting differentiation. Exemplary serum-containing ES medium is made with 80% DMEM (such as Knock-Out DMEM, Gibco), 20% of either defined fetal bovine serum (FBS, Hyclone) or serum replacement (WO 98/30679), 1% non-essential amino acids, 1 mM L-glutamine, and 0.1 mM β-mercaptoethanol. Just before use, human bFGF is added to 4 ng/mL (WO 99/20741, Geron Corp.).

Traditionally, ES cells are cultured on a layer of feeder cells, typically fibroblasts derived from embryonic or fetal tissue. Embryos are harvested from a CF1 mouse at 13 days of pregnancy, transferred to 2 mL trypsin/EDTA, finely minced, and incubated 5 min at 37°C. 10% FBS is added, debris is allowed to settle, and the cells are propagated in 90% DMEM , 10% FBS, and 2 mM glutamine. To prepare a feeder cell layer, cells are irradiated to inhibit proliferation but permit synthesis of factors that support ES cells (∼4000 rads γ-irradiation). Culture plates are coated with 0.5% gelatin overnight, plated with 375,000 irradiated mEFs per well, and used 5 h to 4 days after plating. The medium is replaced with fresh hES medium just before seeding pPS cells.

Scientists at Geron have discovered that pPS cells can be maintained in an undifferentiated state even without feeder cells. The environment for feeder-free cultures includes a suitable culture substrate, particularly an extracellular matrix such as Matrigel® or laminin. The pPS cells are plated at >15,000 cells cm⁻² (optimally 90,000 cm⁻² to 170,000 cm⁻²). Typically, enzymatic digestion is halted before cells become completely dispersed (say, ~5 min with collagenase IV). Clumps of -10 to 2,000 cells are then plated directly onto the substrate without further dispersal. Alternatively, the cells can be harvested without enzymes before the plate reaches confluence by incubating ∼5 min in a solution of 0.5 mM EDTA in PBS. After washing from the culture vessel, the cells are plated into a new culture without further dispersal.

Feeder-free cultures are supported by a nutrient medium containing factors that support proliferation of the cells without differentiation. Such factors may be introduced into the medium by culturing the medium with cells secreting such factors, such as irradiated (∼4,000 rad) primary mouse embryonic fibroblasts, telomerized mouse fibroblasts, or fibroblast-like cells derived from pPS cells. Medium can be conditioned by plating the feeders at a density of ∼5-6 × 10⁴ cm⁻² in a serum free medium such as KO DMEM supplemented with 20% serum replacement and 4 ng/mL bFGF. Medium that has been conditioned for 1-2 days is supplemented with further bFGF, and used to support pPS cell culture for 1-2 days. Alternatively or in addition, other factors can be added that help support proliferation without differentiation, such as ligands for the FGF-2 or FGF-4 receptor, ligands for c-kit (such as stem cell factor), ligands for receptors associated with gp130, insulin, transferrin, lipids, cholesterol, nucleosides, pyruvate, and a reducing agent such as β-mercaptoethanol. Features of the feeder-free culture method are further discussed in International Patent Publication WO 01/51616; and Xu et al., Nat. Biotechnol. 19:971, 2001.

Under the microscope, ES cells appear with high nuclear/cytoplasmic ratios, prominent nucleoli, and compact colony formation with poorly discernable cell junctions. Primate ES cells express stage-specific embryonic antigens (SSEA) 3 and 4, and markers detectable using antibodies designated Tra-1-60 and Tra-1-81 (Thomson et al., Science 282:1145, 1998). Mouse ES cells can be used as a positive control for SSEA-1, and as a negative control for SSEA-4, Tra-1-60, and Tra-1-81. SSEA-4 is consistently present on human embryonal carcinoma (hEC) cells. Differentiation of pPS cells in vitro results in the loss of SSEA-4, Tra-1-60, and Tra-1-81 expression, and increased expression of SSEA-1, which is also found on hEG cells.

### Materials and procedures for preparing hematopoietic cells and their derivatives

Hematopoietic cells are obtained by culturing, differentiating, or reprogramming stem cells in a special growth environment of this invention as defined in the claims that enriches for cells with the desired phenotype (either by outgrowth of the desired cells, or by inhibition or killing of other cell types). These methods are applicable to many types of stem cells, including primate pluripotent stem (pPS) cells described in the previous section.

When derived from an established line of pPS cells, the cell populations and isolated cells will have the same genome as the line from which they are derived. This means that over and above any karyotype abnormalities, the chromosomal DNA will be over 90% identical between the pPS cells and the hematopoietic cells, which can be inferred if the hematopoietic cells are obtained from the undifferentiated line through the course of normal mitotic division. Cells that have been treated by recombinant methods to introduce a transgene or knock out an endogenous gene are still considered to have the same genome as the line from which they are derived (or their progeny), since all non-manipulated genetic elements are preserved.

### Initiating the differentiation process

While not essential to the derivation of hematopoietic cells according to this disclosure, it has been found that an efficient way to perform the derivation is to initiate differentiation in a non-specific way. One method is to cause the pPS cells to form embryoid bodies or aggregates: for example, by overgrowth of a donor pPS cell culture, or by culturing pPS cells in suspension in culture vessels having a substrate with low adhesion properties. Undifferentiated pPS cells are harvested from culture, dissociated into clusters, plated in non-adherent cell culture plates, and cultured in a medium that supports differentiation Reference example
1). In a variation of this method, pPS cells are peeled from the undifferentiated cell culture in strips, which upon culturing in the differentiation medium, aggregate into rounded cell masses Reference example
2). Withdrawing the factors that inhibit differentiation (such as may be present in the conditioned medium used to culture the pPS cells) is part of the differentiation process. In some situations, it can be beneficial to withdraw these factors gradually, for example, by using a medium that has been conditioned with a lower density of feeder cells Reference example
3). Other methods of differentiating pPS cells in a non-specific way are known and may also be suitable for initiating the process of generating hematopoietic cells: for example, by including retinoic acid (RA) or dimethyl sulfoxide (DMSO) in the culture medium; by withdrawing from the usual extracellular matrix upon which the cells are cultured (WO 01/51616), or by forming primitive ectoderm like cells (Rathjen et al., J. Cell Sci. 112:601, 1999).

### Driving differentiation towards hematopoietic cells

In order to drive the culture towards the hematopoietic pathway, undifferentiated pPS cells or initiated cell populations are cultured in a cocktail of hematopoietic differentiation factors as defined in the claims. Alone or in combination, each of the factors may direct cells to differentiate down the hematopoietic pathway, cause outgrowth of cells with a hematopoietic phenotype, inhibit growth of other cell types, or enrich for hematopoietic cells in another fashion: it is not necessary to understand the mechanism of action in order to practice the invention.

Exemplary are combinations of hematogenic cytokines such as stem cell factor (SCF), interleukin 3 (IL-3), interleukin 6 (IL-6), granulocyte-colony-stimulating factor (G-CSF) - either alone, or in combination with bone morphogenic proteins such as BMP-2, BMP-4, or BMP-7. SCF induces an intracellular signal by ligand-mediated dimerization of c-kit, which is a receptor tyrosine kinase related to the receptors for platelet-derived growth factor (PDGF), macrophage colony-stimulating factor (M-CSF), Flt-3 ligand and vascular endothelial growth factor (VEGF). Other factors of interest include Sonic hedgehog (SHH), Delta-1, Jagged-1, and thrombopoietin (TPO). As shown in reference examples 9 and 10, it appears that the cytokines promote formation of the CD45 phenotype (hematopoietic precursor cells), whereas bone morphogenic proteins promote expansion of precursor cells having self-renewal capacity.

Typically, at least two, three, or more than three such factors are combined to create a differentiation cocktail. Human proteins are preferred, but species homologs and variants may also be used. In place of any of these factors, the reader may use other ligands that bind the same receptors or stimulate the same signal transduction pathways, such as receptor-specific antibody. In addition, other components may be included in the medium that neutralizes the effect of other factors that may be present to drive differentiation down a different pathway. An example is antibody to nerve growth factor, which is thought to help minimize the loss of cells in the direction of neurogenic differentiation. The differentiation cocktail is made up in a nutrient medium that supports expansion of the desired cell population, such as a serum-free medium (SF) containing bovine albumin, insulin and transferrin.

The undifferentiated or initiated pPS cells are cultured in the factor cocktail for a sufficient time to permit the desired phenotype to emerge. Selection of the nutrient medium can be important, since some formulations are more supportive of the differentiation process. Inclusion of fetal calf serum in the medium (or its equivalent) enhances the activity of hematopoietic differentiation factors much better than simple mixtures containing only albumin and hormones. In some circumstances, it can also be beneficial to perform this culture over a substrate such as fibronectin supports hematopoietic proliferation.

Contrary to previous predictions, it has been discovered that differentiation of pPS cells into hematopoietic cells can be conducted in a highly efficient manner even in the absence of cocultured stromal cells. Accordingly, this invention includes a method for forming hematopoietic cells in which the differentiated progeny of pPS cells are cultured in the absence of cells that have a different genome, at least until the hematopoietic phenotype emerges in a majority of the population. This means that there are no allotypic or xenotypic cells present in the culture, such as feeder cells, stromal cells, or other cells that provide differentiation factors or a supportive matrix. However, it is permitted to include such cells in the culture medium as an adjunct to the process, except where explicitly excluded. Cells that may enhance the differentiation process include primary stromal cells isolated from human bone marrow, and cells of the MS-5 murine stromal cell line.

Using the techniques disclosed herein, populations of hematopoietic cells have been derived from pPS cells that have an unprecedented proportion bearing a progenitor phenotype. SHH, BMP-4, SCF, IL-3, Flt-3L, and IL-6 in various combinations were able to induce phenotypic and functional hematopoietic progenitors. In reference examples 3 and 4 and example 5, differentiation of pPS cells was initiated by culturing embryoid bodies for 10 days, and then plated in an environment containing 100-300 ng/mL of both SCF and Flt-3L, 10-50 ng/mL of IL-3, IL-6, and G-CSF, 100 ng/mL SHH, and 5-100 ng/mL BMP-4 - all in a medium containing 20% fetal calf serum or in serum-free medium containing albumin, transferring and insulin. After 8 to 15 days, hematopoietic cells emerged that were 8% CD45 +ve, 22% CD34 +ve, and 5.6% double-positive for both markers together. When tested in a CFU assay, the plating efficiency was reproducibly about 1 in 350. In reference examples 9 and 10, the cytokines and BMP-4 were added to the culture the next day after embryoid body formation, further enhancing the proportion of CD45 +ve cells after 15 to 22 days. The presence of BMP-4 allows the user to obtain populations in which 4, 10, or more secondary CFUs form from each primary CFU, indicating the presence of self-renewing hematopoietic progenitors.

### Further maturation pPS derived hematopoietic cells

pPS-derived hematopoietic cells obtained according to the preceding description contain a high proportion of progenitor cells, which are of particular value for therapy of generalized hematopoietic insufficiency, and studying hematopoietic differentiation in vitro.

There are two methods for obtaining mature hematopoetic cells. In one method, the hematopoietic cell populations obtained as already described are further differentiated by culturing in a medium containing appropriate maturation factors. In another method, cell populations that have been initiated into differentiation in a non-specific way are taken directly to the maturation step.

The maturation factors used depend on the ultimate cell type desired. As illustrated in reference example 4, colonies of hematopoietic cells can be generated from embryoid body cells by culturing in an environment containing SCF, GM-CSF, IL-3, and erythropoietin (EPO). This drives the culture towards myeloid cells, resulting in a culture that contains -66% erythroid colonies, -19% monocyte colonies, and -15% granulocyte colonies. Other factors that may be used include G-CSF for granulocytic cells, M-CSF for monocytic cells, IL-2 and IL-4 for lymphoid cells, TPO for megakaryocytes, and EPO for erythroid cells.

### Characteristics of hematopoietic cells

Cells can be characterized according to a number of phenotypic criteria. The criteria include but are not limited to microscopic observation of morphological features, detection or quantitation of expressed cell markers, functional criteria measurable in vitro, and behavior upon infusion into a host animal.

### Phenotypic markers

Cells disclosed herein can be characterized according to whether they express phenotypic markers characteristic of hematopoietic cells of various kinds. Markers of interest include the following:
- Undifferentiated hES cells: SSEA-4, Oct-4
- Primitive hematopoietic cells: CD34, AC133, c-kit, CD38
- Mature multipotent hematopoietic cells: CD45
- Erythroid cells: Glycophorin A
- Early myeloid: CD33
- Monocytic: CD14, CD64, HLA Class II
- Granulocytic: CD13, CD15
- Lymphoid: CD19, immunoglobulin (B cells), CD3 (T cells)
- Megakaryocytic: CD56

Tissue-specific markers can be detected using any suitable immunological technique - such as flow immunocytochemistry for cell-surface markers, or immunohistochemistry (for example, of fixed cells or tissue sections) for intracellular or cell-surface markers. A detailed method for flow cytometry analysis of hematopoietic cells is provided in Gallacher et al., Blood 96:1740, 2000. Expression of a cell-surface antigen is defined as positive if a significantly detectable amount of antibody will bind to the antigen in a standard immunocytochemistry or flow cytometry assay, optionally after fixation of the cells, and optionally using a labeled secondary antibody or other conjugate to amplify labeling.

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. See U.S.

Patent No. 5,843,780 for further details. Sequence data for particular markers listed in this disclosure can be obtained from public databases such as GenBank.

Disclosed herein are hematopoietic cells that are at least 5%, 10%, 20%, or 40% CD34 +ve; 1%, 2%, 5%, or 10% CD45 +ve (or double positive with CD34); 50%, 70%, or 90% positive for CD14, CD14, CD19; and less than 5%, 1%, or 0.2% SSEA-4 +ve or Oct-4 +ve. Various combinations of these features may be present in particular cell populations.

### Functional characteristics

The cells can also be characterized according to functional criteria. See T.A. Bock (Stem Cells 15 Suppl 1:185, 1997) for a review of assay systems for hematopoietic and progenitor cells.

A frequently used test for replicative hematopoietic cells is the ability of such cells to form colonies in a colony forming (CFU) assay. The classic assay is the spleen colony forming assay of Till and McCulloch (Ser. Haematol. 5:15, 1972). Nowadays, colony forming assays are usually run in a methylcellulose matrix supplemented with growth factors. Except where otherwise explicitly required, the definitive CFU assay referred to in this disclosure is conducted as described in reference example 2.

Once the colonies have formed, they can be assessed by morphological criteria and categorized as burst forming unit-erythroid (BFU-E), colony-forming unit-granulocyte-macrophage (CFU-GM), colony-forming unit-megakaryocyte (CFU-M), colony-forming unit-erythroid (CFU-E) and multipotent colonies that make all 4 cell types (CFU-GEMM). Plating efficiency is the ratio of input cells to colonies formed. Hematopoietic cells prepared according to the methods of this invention can have plating efficiencies better than 1 in 2,000, 1 in 500, and under certain circumstances 1 in 100.

Functional criteria of terminally differentiated cells can be determined according to the known characteristics of those cells: for example, the ability of macrophages to phagocytose particles, present antigen, or respond to appropriate cytokines; the ability of granulocytes and platelets to release appropriate mediators; and the ability of lymphocytes to proliferate in response to irradiated allogeneic stimulator cells in a mixed lymphocyte reaction.

### Animal model experiments

Of considerable interest for the purposes of hematopoietic cells for clinical application is the ability of cell populations to reconstitute the hematopoietic system of a host animal. Reconstitution can be tested using several well-established animal models.

Repopulation by administration of hematocompetent cells can be assessed in mice genetically engineered to forestall xenograft rejection. Particularly accommodating is the NOD/SCID mouse, containing the non-obese diabetic (NOD) genotype, crossed into mice with severe combined immunodeficiency (SCID). Use of this model is described in Larochelle et al., Nat. Med. 2:1329, 1996; Dick et al., Stem Cells 15:199, 1997; and Vormoor et al., J. Hematother. 2:215, 1993. Briefly, the mice are sublethally irradiated, and then injected with ∼3 to 4 × 10⁶ CD34 +ve cells through the tail vein. After 8 weeks, bone marrow cells are collected from the femur, tibiae, or iliac crest, and analyzed by surface phenotype and CFU assay for evidence of repopulation with the administered human cells. Since repopulation creates chimerism and a degree of immune tolerance, the hematopoietic cells can be tested in less severely compromised immune systems, such as (in order of increasing rigorousness) nonirradiated NOD/SCID mice, regular SCID mice, nude mice, and immune competent mice.

Further preclinical studies can be conducted in other animal models for hematopoietic potential. A suitable large animal xenograft model is the sheep, which takes advantage of fetal immunologic immaturity and developing spaces in the fetal bone marrow to allow hematopoietic stem cell engraftment without marrow conditioning. This avoids possible stromal abnormalities associated with radiation, chemotherapy, or genetically deficient hosts. In this model, human stem cells colonize and persist in the bone marrow for many years, permitting multilineage differentiation, showing responsiveness to human cytokines, and retaining an ability to engraft into a secondary recipients. See Zanjani et al., Int. J. Hematol. 63:179, 1996; and Zanjani et al., J. Clin. Invest. Med. 93:1051, 1994. Primate models are provided in C.E. Dunbar, J. Intern. Med. 249:329, 2001 and Donahue et al., Hum. Gene Ther. 12:607, 2001. The cell populations of this invention can also be tested in non-human primates by using matched non-human pPS cell preparations to differentiate into hematopoietic cells. See Thomson et al., Proc. Natl. Acad. Sci. USA 92:7844, 1995; and Thomson et al., Biol. Reprod. 55:254, 1996.

### Genetic modification of hematopoietic cells

The hematopoietic cells disclosed herein have a substantial proliferation capacity. If desired, the replication capacity can be further enhanced by increasing the level of telomerase reverse transcriptase (TERT) in the cell, by either increasing transcription from the endogenous gene, or introducing a transgene. Particularly suitable is the catalytic component of human telomerase (hTERT), provided in International Patent Application WO 98/14592. Transfection and expression of telomerase in human cells is described in Bodnar et al., Science 279:349, 1998 and Jiang et al., Nat. Genet. 21:111, 1999. Genetically altered cells can be assessed for hTERT expression by RT-PCR, telomerase activity (TRAP assay), immunocytochemical staining for hTERT, or replicative capacity, according to standard methods. Other methods of immortalizing cells are also contemplated, such as transforming the cells with DNA encoding myc, the SV40 large T antigen, or MOT-2 (U.S. Patent 5,869,243, International Patent Applications WO 97/32972 and WO 01/23555).

Cell populations prepared according to the methods disclosed herein are remarkably free of undifferentiated pPS cells. If desired, the cells can be prepared or further treated to remove undifferentiated cells in vitro, or to safeguard against revertants in vivo. One way of depleting undifferentiated stem cells from the population is to transfect the population with a vector in which an effector gene under control of a promoter that causes preferential expression in undifferentiated cells - such as the TERT promoter or the OCT-4 promoter. The effector gene may be a reporter to guide cell sorting, such as green fluorescent protein. The effector may be directly lytic to the cell, encoding, for example, a toxin, or a mediator of apoptosis, such as caspase (Shinoura et al., Cancer Gene Ther. 7:739, 2000). The effector gene may have the effect of rendering the cell susceptible to toxic effects of an external agent, such as an antibody or a prodrug. Exemplary is a herpes simplex thymidine kinase (*tk*) gene, which causes cells in which it is expressed to be susceptible to ganciclovir (WO 02/42445). Alternatively, the effector can cause cell surface expression of a foreign determinant that makes any cells that revert to an undifferentiated phenotype susceptible to naturally occurring antibody in vivo (GB 0128409.0).

The cells disclosed herein can also be genetically altered in order to enhance their ability to be involved in tissue regeneration, or to deliver a therapeutic gene to the subject being treated. A vector is designed using the known encoding sequence for the desired gene, operatively linked to a promoter that is either constitutive or specifically active in hematopoietic cells. The use of transgenes in genetic therapy is described below.

### Use of hematopoietic precursor cells and their derivatives

This invention provides a growth environment suitable to produce large numbers of hematopoietic precursor cells, and hematopoietic cells of the erythroid, granulocytic, monocyte, megakaryocyte, and lymphoid lineages. These cell populations can be used for a number of important research, development, and commercial purposes.

The cells disclosed herein can be used to prepare a cDNA library relatively uncontaminated with cDNA preferentially expressed in cells from other lineages. The differentiated cells disclosed herein can also be used to prepare monoclonal or polyclonal antibodies that are specific for markers of hematopoietic precursors and their derivatives, according to standard methods.

Of particular interest are use of the compositions for drug development, clinical therapy of hematopoietic pathology, and inducing selective immunotolerance in the context of other types of transplantation therapy.

### Drug screening

Hematopoietic cells disclosed herein can be used to screen for factors (such as solvents, small molecule drugs, peptides, polynucleotides) or environmental conditions (such as culture conditions or manipulation) that affect the characteristics of hematopoietic precursor cells and their various progeny.

In some applications, pPS cells (undifferentiated or differentiated) are used to screen factors that promote maturation into hematopoietic cells, or promote proliferation and maintenance of such cells in long-term culture. For example, candidate maturation factors or growth factors are tested by adding them to cells in different wells, and then determining any phenotypic change that results, according to desirable criteria for further culture and use of the cells.

Other screening applications relate to the testing of pharmaceutical compounds for a potential effect on hematopoietic cell growth, development, or toxicity. Screening may be done either because the compound is designed to have a pharmacological effect on hematopoietic cells, or because a compound designed to have effects elsewhere may have unintended side effects on the hematopoietic system.

The reader is referred generally to the standard textbook *In vitro* Methods in Pharmaceutical Research, Academic Press, 1997, and U.S. Patent 5,030,015. Assessment of the activity of candidate pharmaceutical compounds generally involves combining the differentiated cells of this invention with the candidate compound, either alone or in combination with other drugs. The investigator determines any change in the morphology, marker phenotype, or functional activity of the cells that is attributable to the compound (compared with untreated cells or cells treated with an inert compound), and then correlates the effect of the compound with the observed change.

Cytotoxicity can be determined in the first instance by the effect on cell viability, survival, morphology, and the expression of certain markers and receptors. Effects of a drug on chromosomal DNA can be determined by measuring DNA synthesis or repair. [³H]thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to A. Vickers (pp 375-410 in "In vitro Methods in Pharmaceutical Research," Academic Press, 1997) for further elaboration.

Effect of cell function can be assessed using any standard assay to observe phenotype or activity of hematopoietic cells. Included is an analysis of phenotypic markers and change in the balance of various phenotypes resulting from drug exposure. Also included are colony forming assays and reconstitution assays as described earlier.

### Hematopoietic Reconstitution

Also disclosed herein is the use of hematopoietic precursor cells or their derivatives to restore hematopoietic function in a patient in need of such therapy.

Hematopoietic progenitor cell populations and derivative populations can be used for treatment of acute or chronic hematopoietic dysfunction. Such conditions include inherited or acquired genetic deficiencies of the erythroid, granulocytic, macrophage, megakaryocyte, or lymphoid cell lineage, inadequate hematopoietic capacity causing anemia or immune deficiency, or hematopoietic toxicity. Examples are sickle cell anemia, aplastic anemia, myelodysplastic syndrome, accidental exposure to radiation, and life-threatening autoimmune diseases such as lupus.

Of particular interest is the treatment of cancers, such as leukemias, lymphomas, and certain chemotherapy-sensitive and metastatically active solid tumors, such as myeloma and breast cancer. The patient is subject to myeloablative radiation (1200 cGy) or chemotherapy with agents such as cyclophosphamide, thiotepa, or etoposide - and then reconstituted with the hematopoietic cells of this invention. The ability to grow up large numbers of these cells in advance saves the timing constraints of autologous bone marrow transplantation, and eliminates the risk of reintroducing the malignancy with any resident tumor cells in the autologous cell preparation.

Wherever possible, it is beneficial to match the histocompatibility type of the cells being administered with the histocompatibility type of the patient being treated. Identical matches, or cells that are matched at the HLA-A, HLA-B, and HLA-DR loci are optimal. The availability of a large bank of pPS cell derived hematopoietic progenitors, especially cells homozygous in HLA alleles makes matching easier. Where an exact match is not available, a match at one or two Class I or Class II loci will help. In some such circumstances, further manipulation of the cells may help minimize graft-versus-host disease (GVHD) - such as depletion of T cells from the population to be administered (for example, using antibody against CD2, CD3, or CD4).

The hematopoietic cells are typically prepared for administration as a concentrated cell suspension in a sterile isotonic buffer. Bags of refrigerated or cryopreserved stem cells are thawed to room temperature, and infused through central venous catheters in 20 to 50 mL aliquots. Very roughly, a dose of 3.5 × 10⁶ CD3 +ve cells per kg may be appropriate, depending on the CFU assay plating efficiency. After myeloablation, neutrophil counts may drop below 100 cells/µL, with transfusion-dependant thrombocytopenia of <10,000/µL, and the patient is supported with platelets and matched red blood cells. Engraftment first appears at about day 7 to 21, marked by the observation of neutrophils in the blood and early hematopoietic reconstruction. Once engraftment is established, hematopoietic reconstitution is rapid, with the development of adequate neutrophils (1000/µL) and platelets (20,000/µL) by day 14 to 28. Growth factors such as G-CSF and GM-CSF may augment the therapy.

General approaches to the use of hematopoietic cells and their precursors in clinical medicine are provided in standard textbooks, such as the Textbook of Internal Medicine, 3rd Edition, by W.N. Kelley ed., Lippincott-Raven, 1997; and in specialized references such as Hematopoietic Stem Cell Transplantation, by A.D. Ho et al. eds, Marcel Dekker, 2000; Hematopoietic Cell Transplantation by E.D. Thomas et al. eds., Blackwell Science Inc, 1999; Hematopoietic Stem Cell Therapy, E.D. Ball, J. Lister & P. Law, Churchill Livingstone, 2000.

The use of hematopoietic stem cells in clinical therapy is an evolving field, and other uses will occur to the clinical practitioner. As always, the ultimate responsibility for the use and dosage of the cells of this invention is the responsibility of the physician in charge.

### Gene therapy

The cells disclosed herein can be used not just to reconstitute hematopoietic function, but also to correct or supplement any other deficiency that is amenable to gene therapy. Hematopoietic cells have certain advantages as reservoirs for gene expression: they circulate throughout the body, and regenerate on an ongoing basis. The cells can be genetically modified and tested in vitro before administration, saving the uncertainties of administering a genetic vector to the patient.

To perform genetic therapy, the cells are modified with a transgene comprising the therapeutic encoding region under control of a constitutive or hematopoietic cell specific promoter, using a technique that creates a stable modification - for example, a retroviral or lentiviral vector, or by homologous recombination. The modification can be made on a proliferating culture of hematopoietic cells. Alternatively, the modification can be made while the pPS cells are undifferentiated, and followed by the differentiation paradigm. The cells are then assessed both for hematopoietic function and for expression of the transgene.

After adequate testing, the cells can then be administered to the patient in need of the gene therapy, and then monitored biochemically and clinically for correction of the deficiency. Where the composition is HLA compatible with the subject being treated, there may be no need to myeloablate the patient before treatment, if a mixed population of the patient's own cells and the genetically altered cells provides a sufficient reservoir for expression of the therapeutic gene.

See Murdoch et al. (FASEB J. 15:1628, 2001) for a description of hematopoietic stem cells as novel targets for in utero gene therapy. General references include Stem Cell Biology and Gene Therapy by P.J. Quesenberry et al. eds., John Wiley & Sons, 1998; and Blood Cell Biochemistrytherapy: Hematopoiesis and Gene Therapy (Blood Cell Biochemistry, Vol. 8) by L.J. Fairbairn & N.G. Testa eds., Kluwer Academic Publishers, 1999. These references provide a discussion of the therapeutic potential of stem cells as vehicles for gene therapy; delivery systems for gene therapy, and exemplary clinical applications.

### Cell combinations for inducing specific immune tolerance in regenerative medicine

The cells disclosed herein can also be used to induce immune tolerance to a particular tissue type, in preparation for transplantation of an allograft that is mismatched to the patient. The tolerizing cells are chosen to share histocompatibility markers with the allograft, and are administered to the patient before or during treatment with a cell type that regenerates a cellular function needed by the patient. The resulting immune tolerance subsequently decreases the risk of acute or chronic rejection of the allograft.

Effective cell combinations comprise two components: a first cell type to induce immunological tolerance; and a second cell type that regenerates the needed function. A variety of clinically useful cell types can be derived from pPS cells and other sources for purposes of regenerative medicine.

By way of illustration, neural cells can be generated from pPS cells according to the method described in International Patent Publication WO 01/88104 and application PCT/US02/19477 (Geron Corporation). Undifferentiated pPS cells or embryoid body cells are cultured in a medium containing one or more neurotrophins and one or more mitogens, generating a cell population in which at least -60% of the cells express A2B5, polysialylated NCAM, or Nestin and which is capable of at least 20 doublings in culture. Exemplary mitogens are EGF, basic FGF, PDGF, and IGF-1. Exemplary neurotrophins are NT-3 and BDNF. The proliferating cells can then be caused to undergo terminal differentiation by culturing with neurotrophins in the absence of mitogen. Cell populations can be generated that contain a high proportion of tyrosine hydroxylase positive cells, a characteristic of dopaminergic neurons.

Oligodendrocytes can be generated from pPS cells by culturing them as cell aggregates, suspended in a medium containing a mitogen such as FGF, and oligodendrocyte differentiation factors such as triiodothyronine, selenium, and retinoic acid. The cells are then plated onto a solid surface, the retinoic acid is withdrawn, and the population is expanded. Terminal differentiation can be effected by plating on poly-L-lysine, and removing all growth factors. Populations can be obtained in which over 90% of cells are GalC positive.

Hepatocytes can be generated from pPS cells according to the method described in U.S. Patent 6,458,589 and PCT publication WO 01/81549 (Geron Corporation). Undifferentiated pPS cells are cultured in the presence of an inhibitor of histone deacetylase. In an exemplary method, differentiation is initiated with 1% DMSO (4 days), then 2.5 mM of the histone deacetylase inhibitor n-butyrate. The cells obtained can be matured by culturing 4 days in a hepatocyte culture medium containing n-butyrate, DMSO, plus growth factors such as EGF, hepatocyte growth factor, and TGF-α.

Cardiomyocytes or cardiomyocyte precursors can be generated from pPS cells according to the method provided in PCT/US02/22245. The cells are cultured in a growth environment comprising a cardiotrophic factor that affects DNA-methylation, exemplified by 5-azacytidine. Spontaneously contracting cells can then be separated from other cells in the population, by density centrifugation. Further process steps can include culturing the cells in a medium containing creatine, carnitine, or taurine.

Osteoblasts and their progenitors can be generated from pPS cells according to the method described in PCT/US02/20998. pPS-derived mesenchymal cells are differentiated in a medium containing an osteogenic factor, such as bone morphogenic protein (particularly BMP-4), a ligand for a human TGF-β receptor, or a ligand for a human vitamin D receptor. Cells that secrete insulin or other pancreatic hormones can be generated by culturing pPS cells or their derivatives in factors such as activin A, nicotinamide, and other factors listed in U.S. patent application 60/338,885. Chondrocytes or their progenitors can be generated by culturing pPS cells in microaggregates with effective combinations of differentiation factors listed in U.S. patent application 60/339,043.

To induce tolerance against any such differentiated cells to be grafted into an allogeneic recipient, the patient is pretreated or co-treated with "tolerizing" cells - a population of cells that results in a lower inflammatory or immunological reaction to the allograft cells, as determined by leukocyte infiltration at the injection site, induction of antibody or MLR activity, or increased survival time of the allograft cells. Where the object is to promote allotype-specific tolerance, the tolerizing cells are chosen to be "MHC compatible" with the allograft cells. This means minimally that the tolerizing cells will bear at least one MHC Class I haplotype at the A, B or C locus that is shared with the allograft cells. Increasingly preferred are matches in which the tolerizing cells bear one or both of the A haplotypes and/or B haplotypes of the allograft. In the absence of an exact match, the tolerizing population can be made to contain a plurality of haplotypes of the allograft population by creating a mixture of MHC compatible cells from different lines. It is also possible to tailor the tolerizing cells to the allograft cells exactly, by deriving both cell populations from the same pPS cell line.

The tolerizing cells are pPS derived hematopoietic cells, obtained as described above, and bearing one or more characteristic phenotypic or functional features. Of particular interest are hematopoietic cell populations that contain or can give rise to immunoregulatory T cells, dendritic cells and their precursors, or cells that are capable of forming immunological chimerism upon administration. In an alternative embodiment, the cells used for inducing immune tolerance (or a proportion thereof) still have characteristics of the undifferentiated pPS cells. As illustrated in Reference Examples 6-8, undifferentiated pPS cells appear often to be devoid of substantial MHC Class II antigen. They can actively suppress both an inflammatory response, and an allogeneic and xenogeneic immune response - against themselves, and against third-party stimulator cells.

In certain circumstances, there is a concern that undifferentiated pPS cells or early progenitors may grow or differentiate in an uncontrolled fashion after administration, giving rise to malignancies or other unwanted hyperplasia. There are several options to manage this concern. One approach is to equip the undifferentiated cells with a suicide gene (such as thymidine kinase) that renders the prodrug ganciclovir toxic to the cell (WO 02/42445). After tolerance has been induced, the undifferentiated pPS cells can then be culled from the subject by administering the prodrug. Another approach is to inactivate the undifferentiated pPS cells to an extent that they are no longer capable of proliferation in vivo, but can still perform the activity needed for immunosuppression (Reference Examples 7 & 8). Undifferentiated pPS cells can be inactivated beforehand to inhibit or prevent cell division, by irradiation (∼1000 to 3000 Rads), or by treatment with mitomycin c, or some other inactivating chemotherapeutic, cross-linking, or alkylating agent.

The cell combinations described in this section provide an important new system of regenerative medicine. International Patent Publication WO 02/44343 provides several rodent and non-human primate models for evaluating the viability of tolerizing protocols, and subsequent tissue regeneration.

Treatment of human subjects proceeds by administering the first cell population in such a way to induce tolerance to the second cell population. As an aid to quelling local inflammation, the tolerizing cells can be administered to the same site that will receive the regenerating allograft. Alternatively, as an aid to generating hematopoietic chimerism, the tolerizing cells can be administered systemically. Tolerance induction can be determined by testing the patient's blood lymphocytes in a one-way mixed lymphocyte reaction, using cells of the allograft as stimulators (Reference Example 7). Successful tolerance induction will be demonstrated by reduction in the proliferative response. Hematopoietic chimerism of the recipient can be evaluated by assessing circulating monocytes for HLA type, concurrently with hematopoietic surface markers.

The patient is simultaneously or subsequently administered with compatible neurons, oligodendrocytes, hepatocytes, cardiomyocytes, mesenchymal cells, osteoblasts, hormone-secreting cells, chondrocytes, hematopoietic cells, or some other cell type to treat their condition. After the procedure, they are given the requisite amount of supportive care and monitored by appropriate biochemical markers and clinical criteria for improved function.

For any of the therapeutic purposes described in this disclosure, hematopoietic or immunotolerizing cells are typically supplied in the form of a pharmaceutical composition, comprising an isotonic excipient prepared under sufficiently sterile conditions for human administration. Effective cell combinations can be packaged and distributed separately, or in separate containers in kit form, or (for simultaneous administration to the same site) they can be mixed together and also includes sets of cells that exist at any time during their manufacture, distribution, or use. The cell sets comprise any combination of two or more cell populations described in this disclosure, exemplified but not limited to a type of differentiated pPS-derived cell (hematopoietic cells, neural cells, and so on), in combination with undifferentiated pPS cells or other differentiated cell types, sometimes sharing the same genome or an MHC haplotype. Each cell type in the set may be packaged together, or in separate containers in the same facility, or at different locations, under control of the same entity or different entities sharing a business relationship.

For general principles in formulating cell compositions, the reader is referred to Cell Therapy: Stem Cell Transplantation, Gene Therapy, and Cellular Immunotherapy, by G. Morstyn & W. Sheridan eds., Cambridge University Press, 1996. Compositions and combinations intended for pharmacological distribution and use are optionally packaged with written instructions for a desired purpose, such as the reconstitution of hematopoietic function, genetic therapy, or induction of immune tolerance.

### The following examples are provided as further non-limiting illustrations of particular embodiments of the invention.

### EXAMPLES

### Reference Example 1: Feeder-free propagation of embryonic stem cells

Established lines of undifferentiated human embryonic stem (hES) cells were maintained in a culture environment essentially free of feeder cells.

Conditioned medium prepared in advance using primary mouse embryonic fibroblasts (mEF) isolated according to standard procedures (WO 01/51616).

hES cultures were passaged onto Matrigel® coated plates. About one week after seeding, the cultures became confluent and could be passaged. Cultures maintained under these conditions for over 180 days continued to display ES-like morphology. SSEA-4, Tra-1-60, Tra-1-81, and alkaline phosphatase were expressed by the hES colonies, as assessed by immunocytochemistry, but not by the differentiated cells in between the colonies. Pluripotency was confirmed by subjecting them to established protocols for making particular cell types.

### Reference Example 2: Lack of hematopoietic phenotype in undifferentiated hES cell cultures

Undifferentiated cells of the H1 hES cell line were analyzed by flow cytometry and colony forming (CFU) assay to determine whether any of the characteristics of hematopoietic cells are present in the undifferentiated state.

Cells were harvested from feeder-free culture using either Trypsin-EDTA (1% trypsin, 2% EDTA; Gibco) for 10 min at room temp, or cell dissociation buffer (CDB) for 10 min at 37°C (EDTA and high salt, Gibco). The harvested cells were spun down, resuspended in IMDM (Iscove modified Dulbecco's medium) containing 10% FCS, and then filtered through an 85 µm nylon mesh. They were resuspended in 200 µL PBS containing 3% FCS, and incubated with 2 µL of antibody for 15 min at room temp. The cells were washed twice, and then stained with 15 µL/mL 7AAD (Immunotech) for 15 min at room temp.

**Figure 1** shows the results. The viable cells (gated 7AAD -ve; panel i) were further gated by size (ii) to analyze expression of hematopoietic cell surface markers (iii-vi) in undifferentiated ES cell populations. Events with forward scatter properties below 150 were excluded based on a medium control. Cell percentages are expressed as the mean ± SEM, based on the number of independent experiments (n) indicated at the top of each plot.

Undifferentiated H1 (A, B) and H9 cells (C, D) were analyzed for the expression of various human hematopoietic markers (iii-vi), using quadrants based on the respective isotype controls (inset). None of the cells expressed the human hematopoietic marker CD45, and only 1.2% were CD34 +ve (a marker of primitive human hematopoietic cells; panel iii). The cells were analyzed for expression of other primitive hematopoietic markers, including c-Kit (iv), CD38 (v), and AC133 (v). There was virtually no CD38, but 22-33% were c-Kit +ve, and 13 to 52% were AC133 +ve. 12-38% expressed MHC Class I antigen (HLA-A, B, and C) (vi).

CFU assays were conducted as follows. Undifferentiated hES cells were harvested, and 2 × 10⁵ Trypan Blue negative cells were plated into Methocult™ H4230 methylcellulose (StemCell Technologies Inc., Vancouver BC) containing 50 ng/mL SCF, 10 ng/mL GM-CSF (Novartis), 10 ng/mL IL-3 (Novartis), and 3 U/mL EPO (Amgen). Addition of 25 ng/mL BMP-4 and 300 ng/mL Flt-3L to the growth factor cocktail did not enhance the detection of hematopoietic clonogenic progenitors from the undifferentiated hES cell lines. Cultures were incubated at 37°C with 5% CO₂ in a humidified atmosphere, and monitored for development of colonies for up to 40 days. Colony subtypes were distinguished by their morphological characteristics, and (in the case of the erythroid lineage) a reddish color denoting hemoglobinization. Results are shown in Table 1.

**Table 1: CFU Potential of Undifferentiated hES Cells**

| hES Cell Line | Wells positive for CFU | | No. of CFU | CFU Subtypes |
|---|---|---|---|---|
| H9 (n=3) | 1/6 | = 16.6% | 3 | erythroid |
| H1 (n=4) | 0/9 | = 0% | 0 | (none) |

Undifferentiated hES cells of the H1 line failed to produce hematopoietic colonies in 4 separate experiments, 9 separate wells. Similar results were obtained for undifferentiated H9 cells, with the exception of one experiment in which 3 small erythroid colonies formed.

### Reference Example 3: Hematopoietic phenotype in hES cells cultured with hematopoietic differentiation factors

In this experiment, the H9 line of hES cells was differentiated into hematopoietic progenitors, and the phenotype was assessed by flow cytometry.

Strips of hES cells were formed by traversing the diameter of a confluent 6-well plate with a Pasteur pipette until an accumulation of cells was formed. Each strip was suspended in non-conditioned medium (KO DMEM containing 20% FCS), and cultured for 10 days. At this point, the cultures contained rounded balls of cells, referred to in the subsequent examples as embryoid bodies. Many of the cells were non-viable, as assessed by morphological criteria and trypan blue staining.

Embryoid body cells were harvested, dispersed, and seeded into adherent tissue culture dishes, or fibronectin-coated dishes. The culture medium was BIT medium (BSA, insulin, and transferrin; StemCell Technologies, Vancouver BC), supplemented with 0.1 mM β-mercaptoethanol, 2 mM L-glutamine and the following recombinant human growth factors: 300 ng/mL Stem Cell Factor (SCF, Amgen), 300 ng/mL Flt-3 ligand (Flt-3L, R & D Systems, Minneapolis MN), 50 ng/mL G-CSF (Amgen), 10 ng/mL IL-3 (Novartis, Dorval QC), and 10 ng/mL IL-6 (R & D Systems). Following differentiation, the H9 cells were assessed for expression of hematopoietic cell surface markers by flow cytometry.

**Figure 2** compares the cell surface markers detected on undifferentiated hES cells and their derivatives. Gating strategies employed to properly assess flow cytometric data included the exclusion of debris as defined by forward scatter properties being less than 150 (Panel A i), exclusion of dead and dying cells using the viability stain 7AAD, where positivity for this stain defines those cells to be excluded (Panel A ii), and by defining the quadrants according to the isotype controls (insets). Percentages have been corrected for staining of isotype controls. The undifferentiated cells have no CD45, and 0.1% of the cells are CD34 +ve (Panel A iv). 35% of the undifferentiated H9 cells express AC133 (Panel A v). Primitive hematopoietic cells isolated from bone marrow that are AC133 +ve and CD34 -ve are capable of repopulating immune deficient mice.

Shown below is the analysis of cells differentiated by culturing with SF plus HGF, either in the absence (Panel B) or presence (Panel C) of BMP-4. After differentiation, there is expression of CD45 in 0.9% of the cells, and the primitive surface marker CD34 has increased from 0.1% to 1.5% (Panel B iv). There were no cells expressing both markers. The AC133 +ve cells have been reduced from 35% to 14% (Panel B v). Inclusion of BMP-4 to these serum-free cultures yields cells with a proportion of CD45 +ve cells (0.3%) and CD34 +ve cells (0.2%) similar to undifferentiated hES cells. However, differentiation in the presence of BMP-4 again reduced expression of AC133 (10%; Panel C iv).

### Reference Example 4: Hematopoietic colony formation by differentiated hES cells

**Figure 3** shows the scheme for assessing the hematopoietic capacity of cells differentiated from the H1 line of hES cells. Differentiation was initiated by passaging 3 times in conditioned medium made from mEFs cultured at half the usual density. Strips of cells were then cultured in KO DMEM + 20% FCS to form embryoid bodies, as before. At this point, either the entire contents of the well (containing both the embryoid body cells and dead cells) were harvested, or individual embryoid bodies were isolated, devoid of the dead cells. The harvested cells were assessed by CFU assay (conducted as described in Example 2, with or without BMP-4 which had little observed effect). The cells from the CFU assay were then assessed by flow cytometry for surface phenotype.

**Figure 4** shows the results. The photomicrograph in the upper left corner shows the appearance of a typical culture well in the CFU assay (100x magnification). This culture contained cells capable of massive proliferation and various morphological characteristics reminiscent of macrophage, granulocytic and erythroid type progenitor cells. The small dark patches are dead cells in the assay culture. The oval highlights a cluster of cells demonstrating hemoglobinization (red color), which indicates erythroid cells.

The CFU culture was pooled and stained using primary antibody to glycophorin A (indicating red blood cell precursors); CD45 (indicating hematopoietic cells); CD34, CD38, and AC133 (all indicating primitive human hematopoietic cells; and CD19 (indicating B lymphocytes). Positive staining for CD45 (83-86%) confirmed the presence of hematopoietic cells (Panel A i and ii). Positive staining for glycophorin A (4%) confirmed the presence of erythroid cells (Panel A i). As expected, the glycophorin A positive cells did not stain for CD45. Early hematopoietic progenitors constituted a small percentage of this culture, since 0.7% wereCD34 +ve and 0.2% were AC133 +ve. The CFU culture was devoid of CD19 +ve cells (B lymphocytes), with a small percentage of CD33 +ve cells (0.9%). CD33 is a marker for cells early in the myeloid pathway, distinguished from lymphoid lineages. Since the CFU assay is directed to formation of myeloid progenitors, it is not surprising that no lymphoid cells were observed.

Subtypes of the CFUs in the assay culture is shown in Panel B. The total input into the culture was 20,000 cells, and the total CFU count was 47, which means that the average number of cells it took to form a single colony (the plating efficiency) was 1 in 425.

Flow cytometry was also conducted on individually picked colonies of defined subtype. Two colonies were selected, both having a granulocytic morphology as pictured in Panel C (magnification 50x). The colony was 81-92% CD45 +ve (Panel C i and iv), and 73% CD13 +ve (Panel C i), as expected for a granulocytic colony. The low level of CD15 places it within the hematopoietic hierarchy at the myelocytic stage of development. Primitive markers such as CD34 and c-kit were also found to be present on this colony at 6% and 12% respectively, while AC133 was not expressed.

In order to determine the progenitor contribution of embryoid bodies alone, individual embryoid bodies were isolated from the differentiation culture and assayed for CFUs as before. A total of 50,000 differentiated cells were placed into each assay, and cultured for 11 days prior to assessment

**Figure 5** shows the results. Several CFU subtypes were represented: erythroid cells (100x magnification), granulocytic cells (100x magnification) and macrophages (200x magnification). Quantitative assessment based on the total number of progenitors in the culture (77 colonies) revealed a propensity towards the erythroid lineage, with a plating efficiency of one colony per 649 input cells (Panel B). Two erythroid colonies were analyzed by flow cytometry, and were found to be 93% glycophorin A positive.

### Example 5: Secondary Colony Formation

The presence of secondary progenitors was assayed by picking individual colonies from the CFU assay in the last Example, and replating them into a secondary CFU assays. Two primary colonies from the CFU assay conducted on the entire contents differentiation protocol, and two colonies from the isolated embryoid body differentiation protocol, were each passaged into the secondary CFU assay.

**Figure 6** shows the results. The two granulocytic colonies from the entire contents protocol formed a number of colonies in the secondary assay.

Panel A shows the different secondary colonies derived from one single primary colony of 82,500 cells, showing colonies of granulocytic cells, macrophages, erythroid cells, and a GEMM colony (a mixture of granulocytic, erythroid, macrophage and megakaryocytic cell types). Colony numbers are indicated below. The secondary colonies were harvested and pooled together for flow cytometry. There was a high level of CD45 expression (46%, indicating hematopoietic non-erythroid cells), but low levels of CD34 (Panel A v). The cells in the secondary assay were CD13 +ve (35%; Panel A vi), as was the primary colony from which it was derived. CD14 (indicating monocytes) was low (2%; Panel A vii). Glycophorin A +ve cells were only a small proportion of the pooled assay culture (1.2%; Panel A viii), but erythroid progenitors were clearly present as assessed by morphological criteria.

Panel B shows a secondary colony obtained from a different primary granulocytic colony, consisting of 12,500 cells. Fourteen secondary colonies were obtained in total, all of which were macrophage-like colonies. Flow cytometry of the entire CFU assay population showed that the cells were 50% CD45 +ve , 0.7% CD34 +ve, and 57% CD13 +ve, which indicates the presence of either a monocytic or granulocytic cell type.

The demonstration of secondary colony formation indicates that the original cell was a primitive progenitor with higher proliferative potential than is typical of bone marrow cells forming colonies in a primary CFU assay.

### Reference Example 6: Characterization of MHC expression on undifferentiated hES cells

The expression of MHC antigens on human tissues determines the outcome of allo-specific T cell responses *in vitro* and *in vivo.* MHC Class II is expressed primarily on bone marrow derived cells and thymic epithelium. It presents antigen to the immune system for the purpose of initiating a specific immune response. In contrast, MHC Class I is expressed by virtually all mammalian cells. It plays a role in the effector arm of the immune system, and is recognized by specific T lymphocytes when the host cell is virally infected, histo-incompatible, or otherwise contains a foreign antigen.

MHC expression on undifferentiated hES cells was analyzed by immunostaining and flow cytometry. The hES cell lines used in these studies were: H1 (passages 36 to 45), H7 (passages 37 to 43), and H9 (passages 31 to 40). The following antibodies were used: HLA-A, B, C; HLA-DP, DQ, DR (BD-Pharmingen). Cells were incubated with antibody at 0°C, washed, and counterstained with propidium iodide. Flow cytometric analysis was performed on a FACScan™ or FACScalibur™ flow cytometer (Becton Dickinson).

**Figure 7** shows the results. Grey line indicates MHC antibody staining; the solid line indicates isotype control. The H1, H7, and H9 hES cell lines all express MHC Class I (n = 26), as do human fetal cord blood mononuclear cells (CBMC; n = 4). The hES cells have no detectable MHC Class II (DP, DQ, DR haplotypes), whereas a proportion of the CBMCs express a low level of Class II (second hump). The inset in the final panel shows that treatment of the hES cells with 50-100 units of interferon γ (IFN) still failed to induce detectable expression of MHC Class II.

### Reference Example 7: Immunosuppression by undifferentiated hES cells in culture

The ability of hES cells to induce proliferation of allogeneic T cells was measured in a mixed lymphocyte reaction (MLR). It was found that hES cell lines are unable to induce allo-reactivity in primary human T cells, even after stimulation with IFN-γ.

Peripheral blood mononuclear cells (PBMC) were isolated from heparinized blood using a Ficoll-Hypaque™ density gradient (Amersham Pharmacia), and resuspended in RPMI 1640 medium containing 10% FBS. Alternatively, to enrich for T lymphocytes, separated cells were incubated for 2 h at 37°C, and the non-adherent cells were collected and frozen in 60% AIM-V, 30% fetal bovine serum (FBS), 10% DMSO for later use. Dendritic cells (DCs) were prepared by culturing the remaining adherent cells for 7 d in AIM-V containing 10 ng/ml human recombinant GM-CSF and 10 ng/ml IL-4 (R & D Systems). The mixed lymphocyte reaction was performed as follows: stimulator cells were irradiated (DCs, 3000 Rad; BJ fibroblasts, 3000 Rad; or hES-cell lines, 1000 Rad), and then 1 × 10⁵ to 1 × 10² cells were plated in 96-well round bottom plates in AIM-V medium. Responder PBMC or T cells were added at a concentration of 1 × 10⁵ per well, and the plates were cultured in AIM-V for 5 days. The wells were then pulsed with [³H]thymidine (1 µCi per well) for 16-20 h, harvested, and counted.

**Figure 8** shows the results (mean stimulation index ± SEM of multiple wells from 3 donors). hES cells failed to induce allogeneic T cell proliferation in PBMC responders, while significant T cell proliferation was observed when PBMCs were used as stimulators. Similarly, using fetal blood monocytes as responders, no significant proliferation was seen when hES cells were used as stimulators (Panel A). The lack of T cell stimulating capacity of the hES cell lines H1, H7, and H9 was also seen when T cell enriched (monocyte depleted) PBMCs were used as responders (Panel B). Incubation with IFN-γ caused significant up regulation of MHC class I expression (Inset: gray line = untreated hES cells; dotted line = IFN-γ treated cells; dark line = isotype control). However, hES cell lines H1 and H9 prepared by culturing with IFN-γ to increase MHC expression still failed to stimulate T cell proliferation (Panel C). In related experiments, preparing human foreskin fibroblasts by culturing with IFN-γ made them better able to stimulate T cell.

An inhibition experiment was performed to determine if the undifferentiated hES cells possess an ability to actively modulate the allo-MHC response to third-party stimulator cells. Responder T cells (1 × 10⁵) were cultured for 0 or 2 h with varying numbers of irradiated human fibroblasts and hES cells. Subsequently, 1 × 10⁴ irradiated dendritic cells were added per well. After 5 days culture, the cells were pulsed for 16-20 h with [³H]thymidine, washed, and counted.

**Figure 9** shows the results (mean ± SEM). The hES cells abrogated T cell proliferation stimulated by allogeneic dendritic cells. A vigorous proliferative response was detected when PBMCs were co-cultured with allogeneic professional antigen presenting dendritic cells at a ratio of 10:1. However, addition of any of the undifferentiated hES cell lines to these co-cultures strongly inhibited T cell proliferation *in vitro* (Panel A). Addition of an equivalent number of human fibroblast had no inhibitory effect (Panel A). Serial reduction in the number of hES cells resulted in a gradual loss of the inhibitory effect, showing that inhibition by hES cells of alloactivation in a mixed lymphocyte reaction is dose-dependent (Panel B). The MLR was inhibited at a hES cell:T cell ratio of 1:1 or 1:3.

### Reference Example 8: Lack of allostimulation by undifferentiated hES cells in vivo

The immunogenicity of undifferentiated hES cells was further assessed by testing the capacity of the cells to stimulate a cellular immune response in vivo.

Immune deficient Prk-/- SCID mice were injected intramuscularly with 2 to 5 × 10⁶ undifferentiated hES cells, fetal mononuclear cells, or the MBA-1 human megakaryocyte line. After 48-72 h, tissue was fixed, embedded, and sectioned on a cryostat. Every second section was kept for hematoxylin and eosin (H & E) staining. The presence of leukocytes was identified by their characteristic morphology in H & E-stained sections at 1000 X magnification (analysis done blinded; R > 0.97).

**Figure 10** shows the results of this experiment. Both the MBA-1 cells and the mononuclear cord cells were able to induce a granulocytic infiltration response in the Prk-/- SCID mice. In contrast, no granulocyte infiltration was observed at the injection sites of animals injected with undifferentiated hES cells.

**Figure 11** shows the results of a subsequent experiment using wild type immune competent CD-1 mice. Unlike in the Prk-/- SCID mice, injection of endotoxin containing PBS vehicle induced lymphocyte and granulocyte infiltration at the injection site (bottom left panel). However, injection of vehicle together with hES cells completely abrogated leukocyte infiltration (bottom right panel). Injection of MBA-1 cells resuspended in the same vehicle failed to inhibit leukocyte infiltration (inset).

There are two conclusions from this study. First, the hES cells failed to elicit a response against themselves in either immunodeficient or immunocompetent mice. This suggests that they have the capacity to inhibit what should otherwise be a xenogeneic response. Administering cells to a xenogeneic host is in principle a more rigorous test than administering them to an allogeneic human, because of the much higher level of antigen mismatch. Second, the hES cells apparently were also able to inhibit the non-specific infiltration that otherwise occurs in response to endotoxin - an inflammatory response that is not antigen-specific.

As indicated earlier in this disclosure, the ability of undifferentiated hES cells to actively inhibit both immune and inflammatory reactions has important implications for clinical therapy.

### Reference Example 9: BMP promotes self-renewal of hES cell derived hematopoietic progenitors

In the next series of experiments, hematopoietic cells were obtained from hES cells using a modified differentiation timeline.

Undifferentiated hES cells in feeder-free culture were treated with Collagenase IV and scraped off the Matrigel® matrix in strips. They were then transferred to low attachment plates, and embryoid bodies formed overnight in differentiation medium containing 20% non-heat inactivated FBS. The medium was changed the very next day to medium containing either hematopoietic cytokines (300 ng/mL SCF; 300 ng/mL Flt-3 ligand, 10 ng/mL IL-3, 10 ng/mL IL-6, and 50 ng/mL G-CSF); or BMP-4 (50 ng/mL); or both cytokines and BMP-4. Control cultures continued in the same differentiation medium without any added factors. Media were changed every 3 days.

**Figure 12** shows the total cell count and number of CD45 +ve hematopoietic progenitor cells that were obtained. Also shown is the number of primary CFUs obtained per 10⁵ input cells. Cytokines considerably improved the yield of CD45 +ve cells (p < 0.02) and CFU (p < 0.001) compared with control. By any of these criteria, there was negligible effect of BMP-4, either with or without the cytokines.

**Figure 13** shows the results of secondary CFUs, emphasizing the importance of BMP-4. Self-renewal of hematopoietic progenitors derived from hES cells under control conditions was an infrequent event, occurring from only 6% of primary CFU (Left Panel). In contrast, treatment of differentiating hES cells with cytokines enhanced the self-renewal capacity to 21% of all primary CFU examined. While the frequency of progenitor self-renewal increased when the cells were differentiated with cytokines, the magnitude of self-renewal from both control or cytokine derived hematopoietic progenitors was minimal, with an average of 0.5 and 0.3 secondary CFU detected per primary CFU respectively (Right Panel). When hES cells were differentiated with both cytokines and BMP-4, 36% of primary CFU generated secondary CFUs. Individual primary CFU arising from hES cells differentiated in the presence of cytokines plus BMP-4 generated up to 4 secondary CFU per primary CFU, a magnitude of self-renewal 8-fold higher than control or cytokine treatment alone. Although treatment of differentiating hES cells with BMP-4 alone did not enhance hematopoietic specification above basal potential (Example 2 and 3), BMP-4 was shown in this example to influence self-renewal potential of primary hematopoietic progenitors. Greater than 50% of primary CFU generated in the presence of BMP-4 were capable of self-renewal (Left Panel), with an average capacity to form up to 10 secondary CFUs per primary CFU (Right Panel), a 20-fold increase in self renewal capacity over control or cytokine differentiated cells.

To compare the frequency and magnitude of progenitor self-renewal between hES-derived hematopoietic progenitors and known sources of committed hematopoietic tissue, primary CFU arising from human cord blood samples were assayed for self-renewal capacity in the same way (Right Panel, inset). Primary CFU derived from cord blood did not give rise to secondary progenitors when assayed individually. However, when multiple primary colonies were pooled, progenitor self-renewal was observed at a frequency of 0.5 secondary CFU per primary CFU. This shows the rarity of self-renewing progenitors from committed hematopoietic tissue, compared with hematopoietic progenitors derived from hES cells differentiating in the presence of BMP-4.

These results demonstrate that differentiating hES cells in the presence of BMP-4 produces hematopoietic progenitors that possess superior self-renewal capacity.

### Reference Example 10: Kinetics of progenitor induction

In this example, the kinetics of hematopoietic cell differentiation were examined further. The cells were cultured with HGF Cytokines and BMP-4, beginning the day after embryoid body formation. Cells were sampled at various times in the culture, and analyzed for CD45 and primary CFUs

**Figure 14** shows the results. No hematopoietic cells were observed at Day 3, 7, or 10 of culture with cytokines plus BMP-4. The frequency of CD45 +ve cells increased considerably on Day 15 and Day 22. At Day 7 and 10, clonogenic efficiencies in the CFU assay was below 1 in 15,000, but rose to 1 in 262 on Day 15. The increase in clonogenic efficiency between Day 15 and Day 22 was not statistically significant, suggesting that the proliferation of committed hematopoietic cells between Days 15 and 22 occurs concomitantly with differentiation and loss of progenitor function.

This disclosure proposes a conceptual model regarding directed hematopoietic differentiation of hES cells. The model is offered solely to enhance the reader's appreciation of the underlying process; it is not meant to limit the invention where not explicitly required.

The generation of hematopoietic progeny from hES cells seems to occur in two phases - an induction phase governed by programs initiated by hematopoietic cytokines, followed by a proliferative phase of committed hematopoietic cells. The cytokines induce committed hematopoietic progenitors capable of multilineage maturation, represented by the CD45 marker. Few committed hematopoietic progenitors arising from spontaneous differentiation of hES cells under control conditions were capable of self-renewal in the secondary CFU assay, and are therefore probably terminally differentiated. Thus, intrinsic programs governing hES cell differentiation fail to generate maintenance capacity that is induced with cytokine and BMP-4 treatment.

The results show that BMP-4 (either alone or in combination with cytokines) has no effect on the frequency or total number of hematopoietic progenitors obtained from hES cells. However, derivation of hES cells in the presence of BMP-4 gives rise to unique hematopoietic progenitors possessing greater self-renewal capacity. BMP-4 may confer its effect during the first 14 days of development, stimulating long-term programs responsible for progenitor renewal.

## Claims

1. A growth environment comprising a culture medium and a substrate on a solid surface for differentiating pPS cells into hematopoietic cells, wherein the culture medium contains a mixture of hematopoietic growth factors, chosen from two or more of: stem cell factor, interleukin-3, interleukin-6, granulocyte-colony stimulating factor, BMP-2, BMP-4, BMP-7, sonic hedgehog, Delta-1, Jagged-1 and thrombopoietin, wherein no stromal cells are present.

2. The growth environment of claim 1, wherein the mixture of hematopoietic growth factors comprises at least two of the following: stem cell factor (SCF), FLT-3 ligand, IL-3, IL-6, and granulocyte colony stimulating factor (G-CSF).

3. The growth environment of claim 1 or claim 2, which is suitable for producing hematopoietic progenitors, erythroid cells, granulocytic cells, monocytic cells, megakaryocytes, or lymphoid cells.

4. The growth environment of any of claims 1 to 3, wherein the pPS cells are human embryonic stem cells or cultured from a human blastocyst.

## Patentansprüche

1. Kultivierungsumgebung, umfassend ein Kulturmedium und ein Substrat auf einer festen Oberfläche zur Differenzierung von pPS-Zellen zu hämatopoetischen Zellen, wobei das Kulturmedium ein Gemisch von hämatopoetischen Wachstumsfaktoren, die aus zwei oder mehr von Stammzellfaktor, Interleukin-3, Interleukin-6, Granulozyten-Koloniestimulierungsfaktor, BMP-2, BMP-4, BMP-7, Sonic Hedgehog, Delta-1, Jagged-1 und Thrombopoetin gewählt sind, enthält, wobei keine Stromazellen vorliegen.

2. Kultivierungsumgebung nach Anspruch 1, wobei das Gemisch von hämatopoetischen Wachstumsfaktoren wenigstens zwei der Folgenden umfasst: Stammzellfaktor (SCF), FLT-3-Ligand, IL-3, IL-6 und Granulozyten-Koloniestimulierungsfaktor (G-CSF).

3. Kultivierungsumgebung nach Anspruch 1 oder Anspruch 2, die sich zur Herstellung von hämatopoetischen Vorläuferzellen, erythroiden Zellen, granulozytischen Zellen, monozytischen Zellen, Megakaryozyten oder lymphoiden Zellen eignet.

4. Kultivierungsumgebung nach einem der Ansprüche 1 bis 3, wobei es sich bei den pPS-Zellen um menschliche embryonale Stammzellen handelt oder die pPS-Zellen von einer menschlichen Blastozyste kultiviert werden.

## Revendications

1. Environnement de croissance comprenant un milieu de culture et un substrat sur une surface solide pour différencier des cellules pPS en cellules hématopoiétiques, dans lequel le milieu de culture contient un mélange de facteurs de croissance hématopoiétiques, choisis parmi deux ou plus de deux parmi : un facteur de cellule souche, l'interleukine 3, l'interleukine 6, le facteur stimulant les colonies de granulocytes, BMP-2, BMP-4, BMP-7, sonic hedgehog, Delta-1, Jagged-1 et la thrombopoïétine, dans lequel aucune cellule stromale n'est présente.

2. Environnement de croissance de la revendication 1, dans lequel le mélange de facteurs de croissance hématopoïétiques comprend au moins deux des suivants : facteur de cellules souches (SCF), ligand de FLT-3, IL-3, IL-6 et facteur stimulant les colonies de granulocytes (G-CSF).

3. Environnement de croissance de la revendication 1 ou la revendication 2, qui est adapté pour produire des progéniteurs hématopoiétiques, des cellules érythroïdes, des cellules granulocytaires, des cellules monocytaires, des mégacaryocytes ou des cellules lymphoïdes.

4. Environnement de croissance de l'une quelconque des revendications 1 à 3, dans lequel les cellules pPS sont des cellules souches embryonnaires humaines ou cultivées à partir d'un blastocyste humain.
